# EUROPEAN PATENT SPECIFICATION

(11) **EP 2 971 175 B1**
(45) Date of publication and mention of the grant of the patent: **17.04.2019**
(21) Application number: 14774536.8
(22) Date of filing: 13.03.2014
(51) Int. Cl.: C12Q 1/68, C12P 19/34, C12N 9/24

(54) **MINIMIZING ERRORS USING URACIL-DNA-N-GLYCOSYLASE**
FEHLERMINIMIERUNG MITTELS URACIL-DNA-N-GLYCOSYLASE
MINIMISATION D'ERREURS UTILISANT DE L'URACIL-ADN-N-GLYCOSYLASE

(30) Priority: 14.03.2013 US 201361782698 P
(43) Date of publication of application: 20.01.2016
(73) Proprietor: Abbott Molecular Inc., Des Plaines, IL 60018 (US)
(72) Inventor: SHAH, Ankur, Des Plaines Illinois 60018 (US); CHOI, Won, Des Plaines Illinois 60018 (US)
(74) Representative: Chapman, Desmond Mark
(86) International application number: PCT/US2014/026163
(87) International publication number: WO 2014/160254

(56) References cited:
- WO-A1-2011/106315
- WO-A2-2007/120627
- US-A1- 2007 117 114
- M Pruvost: "INTRODUCTION", BioTechniques, 1 January 2005 (2005-01-01), XP055292220, Retrieved from the Internet: URL:http://www.biotechniques.com/multimedi a/archive/00004/BTN_A_05384ST03_O_4209a.pd f [retrieved on 2016-07-29]
- H. YANG ET AL: "Characterization of a Thermostable DNA Glycosylase Specific for U/G and T/G Mismatches from the Hyperthermophilic Archaeon Pyrobaculum aerophilum", JOURNAL OF BACTERIOLOGY, vol. 182, no. 5, 1 March 2000 (2000-03-01) , pages 1272-1279, XP055292145, US ISSN: 0021-9193, DOI: 10.1128/JB.182.5.1272-1279.2000
- DO ET AL.: 'Dramatic reduction of sequence artefacts from DNA isolated from formalin-fixed cancer biopsies by treatment with uracil- DNA glycosylase.' ONCOTARGET vol. 3, no. 5, May 2012, pages 546 - 548, XP055282871

## Description

### FIELD OF INVENTION

Provided herein is technology relating to enzymatic modification of nucleic acids and particularly, but not exclusively, to methods, kits, and compositions relating to using uracil-DNA-N-glycosylase for minimizing or eliminating errors in a DNA sequence due to deamination of cytosine residues.

### BACKGROUND

Changes in pH, temperature, ionic strength, pressure, etc. are often used in molecular biology processes and assays to effect changes in sample components such as nucleic acids, proteins, cofactors, etc. However, some molecular biological manipulations of particular sample components (e.g., a particular protein) produce undesirable effects on other sample components (e.g., a nucleic acid). For example, the use of a heat-activated enzyme for molecular biology requires a period of heating (e.g., 10 to 20 minutes at 95°C) to activate the enzyme. During this heating, nucleic acids (e.g., DNA) present in the sample are also heated. Heating a nucleic acid induces deamination of cytosine (see, e.g., Lindahl and Nyberg (1974) "Heat-induced deamination of cytosine residues in deoxyribonucleic acid, Biochemistry 13(16): 3405), which results in converting the cytosine base to a uracil base. Whereas a cytosine base pairs with a guanine, a uracil base pairs with an adenine. As such, the uracil base codes for an adenine during synthesis of a complementary DNA strand. This initial error in a DNA strand results in a G to A mutation and/or a C to T mutation in the strands of DNA subsequently synthesized from the damaged template.

While a single-stranded DNA molecule with 2 million bases will experience a single deamination event involving cytosine every 2.8 hours at pH 7.4 and 37°C, a 95°C incubation of DNA induces deamination of cytosine at a rate that is approximately 2 × 10⁻⁷ deamination events per second (see, e.g., Lindahl and Nyberg, supra). Based on this rate, heating DNA for 10 minutes at 95°C causes conversion of a cytosine to a uracil in approximately 1 out of every 8333 cytosines. Accordingly, this rate is relevant since molecular biological samples often comprise more than a million (or even more than a billion) cytosine residues. This conversion is a problem for single nucleotide polymorphism (SNP) detection assays in which the SNPs targeted for detection are either G to A or C to T conversions. For example, approximately 1 in 8333 copies of a wild-type sequence would convert to a mutant copy during heat activation and thus generate a false positive result that the SNP was present in the sample. As a result, technologies are needed to address the thermal deamination of nucleic acids in molecular biological processes.

WO 2007/120627 discusses methods and compositions for repairing a polynucleotide using a DNA ligase, at least one endonuclease as well as a cofactor selected from NAD+ or ATP. Pruvost *et al.* 2005 discusses minimising the DNA contamination in ancient DNA samples using a uracil-N-glucosylase. WO 2011/106315 discusses automated processes for isolating, amplifying, and analyzing a target nucleic acid sequence that may be present in a fluid test sample.

### SUMMARY

Accordingly, disclosed herein is technology relating to enzymatic modification of nucleic acids and particularly, but not exclusively, to methods and compositions relating to using uracil-DNA-N-glycosylase (a "UDG" enzyme, typically encoded by a gene named UNG) to minimize or eliminate errors in a DNA sequence due to deamination of cytosine residues (e.g., as a result of heating DNA). Uracil-DNA glycosylases prevent the fixation of C to U and G to A mutations into replicated DNA by eliminating uracil from DNA molecules before they can serve as templates for DNA synthesis. The uracil-DNA-N-glycosylase cleaves the N-glycosylic bond connecting the uracil base to the DNA backbone by flipping the damaged base out of the double helix and cleaving the N-glycosidic bond, thus removing the damaged nitrogenous base and leaving the sugar-phosphate backbone intact. As a result of this process, an abasic site (also called an apurinic/apyrimidinic site or an AP site) is produced in the DNA strand.

Abasic sites cause polymerases to stall. Under some conditions, abasic sites produce breaks in a DNA strand (e.g., spontaneously and/or from the action of an enzyme that cleaves nucleic acids at an abasic site). In either case, a polymerase does not proceed past the abasic site and thus does not introduce a base in a complementary strand opposite the abasic site. As a result, the polymerase does not produce a mutant DNA strand complementary to a damaged DNA template, that results from priming by a damaged oligonucleotide primer, or as the result of other damaged nucleic acids in various molecular biological methods. Thus, the damaged base does not cause a proliferation of mutant sequences in a sample. Without this activity, the U resulting from the damaged C guides a polymerase to incorporate an A rather than a G in the opposing complementary strand during DNA synthesis; subsequent rounds of synthesis incorporate a T opposite the errant A. As a result, the initial deamination event results in fixation of the C to T and G to A mutation in all subsequent copies. Deamination of C bases thus results in mutations in natural populations.

In addition, the deamination of C bases to produce U bases is significantly problematic in methods that synthetically amplify nucleic acids, e.g., in a polymerase chain reaction (PCR), a ligase chain reaction, a primer extension reaction, and in other amplification reactions as described in more detail below. As an example, during a PCR the amplification of DNA occurs exponentially. As a result, minor events representing a small proportion of the nucleic acids in a sample, such as the deamination of a single C base in a single DNA strand, are amplified and represented in significant amounts during the PCR and in the end product of the PCR. In addition, plateau effects in the later cycles of a PCR result in the overrepresentation of initially minor species of nucleic acids in the final amplified product. These deamination problems are exacerbated by the periods of heating during PCR-associated thermocycling that are known to induce deamination of C bases.

Accordingly, the technology disclosed herein relates to enzymes that recognize and remove U bases from DNA. In some embodiments the enzyme is isolated from a heat stable organism. Heat stable enzymes are produced by a number of processes and the technology is not limited by the source of the heat stable enzyme. For instance, in some embodiments the heat stable enzyme is an enzyme isolated from a thermophilic organism (e.g., a thermophilic member of the Archaea such as *Archaeglobus fulgidis*).

The technology encompasses compositions, methods, and reaction mixtures comprising (or comprising use of) a native heat-stable enzyme that recognizes and removes U bases from DNA, e.g., a native heat-stable uracil-DNA-N-glycosylase; a recombinant heat-stable enzyme that recognizes and removes U bases from DNA, e.g., a recombinant heat-stable uracil-DNA-N-glycosylase, a wild-type heat-stable enzyme that recognizes and removes U bases from DNA, e.g., a wild-type heat-stable uracil-DNA-N-glycosylase; a mutant heat-stable enzyme that recognizes and removes U bases from DNA, e.g., a mutant heat-stable uracil-DNA-N-glycosylase; and/or an engineered heat-stable enzyme that recognizes and removes U bases from DNA, e.g., an engineered heat-stable uracil-DNA-N-glycosylase.

In some embodiments, the enzyme is isolated from a mesophilic organism and is a heat-stable enzyme. In some embodiments, a heat stable enzyme is produced from a less heat-stable enzyme by methods such as random mutagenesis, in silico modeling, rational (directed) mutagenesis, rational (directed) enzyme design, in vitro evolution (e.g., SELEX), etc. In some embodiments, the enzyme is a cold-stable enzyme (e.g., isolated from a psychrophilic or cryophilic organism) that is also heat-stable. In some embodiments, the enzyme is a uracil-DNA-N-glycosylase (a "UDG" or an "UNG").

Heat-stable uracil-DNA-N-glycosylases are commercially available. In addition, the nucleotide sequences and/or amino acid sequences of several heat-stable uracil-DNA-N-glycosylases are known and the genome sequences of many organisms, including many thermophiles, are known. As such, one can purchase a heat-stable uracil-DNA-N-glycosylase or isolate a heat-stable uracil-DNA-N-glycosylase from an organism based on known nucleotide and/or protein sequences and available genome sequences, e.g., by PCR (e.g., using primers targeting conserved regions, using degenerate primers), probe methods, or by complete synthesis in vitro. Thermophiles are distinguishable from mesophiles by characteristics such as their optimal growth temperature (e.g., which is higher than the optimal growth temperature of a mesophile) and genomic characteristics (e.g., such as a higher GC content).

As such, in some embodiments the technology includes methods and compositions related to a heat-stable UDG that retains its activity during periods of heating, e.g., during the heating steps of a PCR, the heating steps of a sequencing reaction, the heating steps of sample preparation, and/or during incubation at high temperature, e.g., at 60°C or more, 70°C or more, 80°C or more, at 90°C or more, or at 95°C or more. For example, some PCR methods use a heat-activated polymerase such as a heat-activated Taq polymerase. Often, the polymerase remains inactive until heated at 95°C or more. During this period of heating, deamination of C bases occurs.

The technology is broadly applicable to minimize or eliminate sequence errors in a nucleic acid due to deamination of cytosines, e.g., as a result of any heating of the nucleic acid in a sample. Heating of samples is often performed in molecular biological techniques used to prepare samples, e.g., to isolate and prepare nucleic acids and other biomolecules. For example, the preparation of nucleic acids from formalin-fixed paraffin-embedded samples (FFPE samples) is often associated with periods of heating that produce cytosine deamination and associated errors in the sequences of the nucleic acids isolated from the FFPE samples. As another example, heating is often used to prepare nucleic acids for sequencing (e.g., in the preparation of sequencing libraries) and during sequencing reactions themselves. Accordingly, the technology is applicable to extant sequencing technologies and sequencing technologies yet to be developed, e.g., preparation methods and protocols associated with Sanger and Maxam sequencing, Second Generation (a.k.a. Next Generation or Next-Gen), Third Generation (a.k.a. Next-Next-Gen), or Fourth Generation (a.k.a. N3-Gen) sequencing technologies including, but not limited to, pyrosequencing, sequencing-by-ligation, single molecule sequencing, sequence-by-synthesis (SBS), massive parallel clonal, massive parallel single molecule SBS, massive parallel single molecule real-time, massive parallel single molecule real-time nanopore technology, methods using zero mode waveguides, etc. Morozova and Marra provide a review of some such technologies in Genomics, 92: 255 (2008). Exemplary technologies are methods that include amplification steps such as pyrosequencing commercialized by Roche as the 454 technology platforms (e.g., GS 20 and GS FLX), the Solexa platform commercialized by Illumina, and the Supported Oligonucleotide Ligation and Detection (SOLiD) platform commercialized by Applied Biosystems. Other approaches, also known as single-molecule sequencing, are exemplified by the HeliScope platform commercialized by Helicos BioSciences, and emerging platforms (e.g., nanopore sequencing, pH-based detection of nucleotide incorporation events, Xpandomer technologies) commercialized by VisiGen, Oxford Nanopore Technologies Ltd., Life Technologies/Ion Torrent, and Pacific Biosciences, respectively.

The technology is also related to methods associated with sample preparation such as restriction digestion, genome amplification, fragmentation, end-repair, ligation (e.g., linker ligation), strand separation, melting of secondary and/or tertiary structures, contaminant removal, protein removal, cell lysis, preparation of nucleic acids from tissue and/or cells and/or biological fluids, attachment to a solid support, primer annealing, etc.

The technology also finds use in microarray technologies, probe detection technologies (e.g., blotting methods such as Southern blotting, Northern blotting, dot-blot, slot-blot, hybridization protection assays, etc.).

Methods of nucleic acid amplification often incorporate heating of samples. Examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (e.g., PCR) require that RNA be reversed transcribed to DNA prior to amplification (e.g., RT-PCR), whereas other amplification techniques directly amplify RNA (e.g., TMA and NASBA).

The polymerase chain reaction (U.S. Pat. Nos. 4,683,195, 4,683,202, 4,800,159 and 4,965,188), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR see, e.g., U.S. Pat. Nos. 4,683,195, 4,683,202 and 4,800,159; Mullis et al., Meth. Enzymol. 155: 335 (1987); and, Murakawa et al., DNA 7: 287 (1988.

Transcription mediated amplification (U.S. Pat. Nos. 5,480,784 and 5,399,491), commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. See, e.g., U.S. Pat. Nos. 5,399,491 and 5,824,518. In a variation described in U.S. Publ. No. 20060046265 , TMA optionally incorporates the use of blocking moieties, terminating moieties, and other modifying moieties to improve TMA process sensitivity and accuracy.

The ligase chain reaction (Weiss, R., Science 254: 1292 (1991), commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded ligated oligonucleotide product.

Strand displacement amplification (Walker, G. et al., Proc. Natl. Acad. Sci. USA 89: 392-396 (1992); U.S. Pat. Nos. 5,270,184 and 5,455,166), commonly referred to as SDA, uses cycles of annealing pairs of primer sequences to opposite strands of a target sequence, primer extension in the presence of a dNTPaS to produce a duplex hemiphosphorothioated primer extension product, endonuclease-mediated nicking of a hemimodified restriction endonuclease recognition site, and polymerase-mediated primer extension from the 3' end of the nick to displace an existing strand and produce a strand for the next round of primer annealing, nicking and strand displacement, resulting in geometric amplification of product. Thermophilic SDA (tSDA) uses thermophilic endonucleases and polymerases at higher temperatures in essentially the same method (EP Pat. No. 0 684 315).

Other amplification methods include, for example: nucleic acid sequence based amplification (U.S. Pat. No. 5,130,238), commonly referred to as NASBA; one that uses an RNA replicase to amplify the probe molecule itself (Lizardi et al., BioTechnol. 6: 1197 (1988), commonly referred to as Qβ replicase; a transcription based amplification method (Kwoh et al., Proc. Natl. Acad. Sci. USA 86:1173 (1989)); and, self-sustained sequence replication (Guatelli et al., Proc. Natl. Acad. Sci. USA 87: 1874 (1990),). For further discussion of known amplification methods see, e.g., Persing, David H., "In Vitro Nucleic Acid Amplification Techniques" in Diagnostic Medical Microbiology: Principles and Applications (Persing et al., Eds.), pp. 51-87 (American Society for Microbiology, Washington, DC (1993)).

In some embodiments, amplification is isothermal amplification method. In some embodiments, amplification methods are solid-phase amplification, polony amplification, colony amplification, emulsion PCR, bead RCA, surface RCA, surface SDA, etc., as will be recognized by one of skill in the art. In some embodiments, amplification methods that result in amplification of free DNA molecules in solution or tethered to a suitable matrix by only one end of the DNA molecule are used. In some embodiments, methods that rely on bridge PCR, where both PCR primers are attached to a surface (see, e.g., WO 2000/018957, U.S. 7,972,820; 7,790,418 and Adessi et al., Nucleic Acids Research (2000): 28(20): E87) are used. In some cases the methods of the disclosed herein can create a "polymerase colony technology", or "polony", referring to a multiplex amplification that maintains spatial clustering of identical amplicons. These include, for example, in situ polonies (Mitra and Church, Nucleic Acid Research 27, e34, Dec. 15, 1999), in situ rolling circle amplification (RCA) (Lizardi et al., Nature Genetics 19, 225, July 1998), bridge PCR (U.S. Pat. No. 5,641,658), picotiter PCR (Leamon et al., Electrophoresis 24, 3769, November 2003), and emulsion PCR (Dressman et al., PNAS 100, 8817, Jul. 22, 2003).

The technology is not limited in the type (e.g., with regard to its size, purpose, origin (e.g., natural or synthetic), etc.) of nucleic acid that is damaged (e.g., by deamination of cytosine (e.g., heat-induced or other (e.g., pH-induced) deamination of cytosine) or incorporation of uracil in DNA) and subsequently exposed to an enzyme that removes uracil from a nucleic acid (e.g., a UDG). For example, in some procedures a probe, oligonucleotide, primer, linker, genome, amplicon, plasmid, or other nucleic acid is damaged, e.g., it comprises a deaminated cytosine, e.g., as a result of heating. The technology encompasses minimizing or eliminating errors that arise from these types of damaged nucleic acids.

As such, the technology relates to a heat stable UDG that retains activity during and after an incubation at high temperature to activate a polymerase (e.g., a 10-minute incubation at 95°C to activate a Taq polymerase). Furthermore, the technology relates to a heat stable UDG that is active at a high temperature to prevent primer annealing and strand extension during PCR. In particular, the UDG removes U bases from the DNA before a polymerase places an adenine opposite the uracil. The technology is not limited in the UDG that finds use in the related compositions, methods, and uses. As such, any heat stable UDG or enzyme with similar activity finds use in the technology. As such, the technology provides methods for increasing sensitivity and reducing false positive rates in molecular biological assays that relate to ascertaining a DNA sequence. In particular, the technology provides methods for increasing sensitivity and reducing false positive rates in molecular biological assays that detect a G to A and/or a C to T mutation such as in a PCR-based SNP detection assay.

Embodiments of the technology include kits for using uracil-DNA-N-glycosylase for minimizing or eliminating errors in a DNA sequence due to deamination of cytosine residues. Kit embodiments comprise one or more vessels (e.g., vials, ampules, bottles, packets, and the like) containing a heat-activated polymerase (e.g., a polymerase for PCR, e.g., real-time PCR) and a heat-stable enzyme that recognizes and removes U bases from DNA, e.g., a heat-stable uracil-DNA-N-glycosylase. Some embodiments include an enzyme that cleaves a nucleic acid at an abasic site (e.g., a nuclease, e.g., a heat-stable nuclease). In some embodiments, a single enzyme provides both the uracil-removal activity and the nuclease activity; in some embodiments one enzyme provides the uracil-removal activity and a second enzyme provides the nuclease activity.

In some embodiments of said kits, one composition comprises a mixture of the two enzymes (e.g., a heat-active polymerase and a heat-stable enzyme that recognizes and removes U bases from DNA) and some embodiments of said kits comprise two compositions, one that comprises a heat-active polymerase and a second that comprises a heat-stable enzyme that recognizes and removes U bases from DNA. In kits that comprise two compositions, the two compositions may be mixed together before use, e.g., in a defined proportion described by a protocol provided with the kit. Further embodiments of kits comprise a control nucleic acid, e.g., for embodiments of kits that find use in detecting mutations in nucleic acids (such as an assay to determine the presence of a SNP). Examples of control nucleic acids are a nucleic acid that comprises the wild-type sequence at the SNP location (e.g., a negative control) and one or more nucleic acids that comprise a mutant sequence at the SNP location (e.g., a positive control). Kit embodiments may also comprise a nucleic acid that is unrelated to the nucleic acid that is the subject of the assay, e.g., a nucleic acid that serves as an internal reference control for normalizing the amount of amplicon produced or activity of the UDG enzyme. For instance, some control nucleic acids are synthetic DNA molecules comprising one or more uracil bases to provide a positive control of UDG activity.

The technology encompasses compositions that are reaction mixtures. For example, embodiments of the technology include a reaction mixture comprising a heat-activated polymerase and a heat-stable enzyme that recognizes and removes U bases from DNA, e.g., a heat-stable uracil-DNA-N-glycosylase. In some embodiments, the heat-activated polymerase is a heat-stable polymerase. In some embodiments, the reaction mixture comprises an actively polymerizing heat-activated, heat-stable polymerase, e.g., a polymerase that is adding nucleotides to a strand of a nucleic acid in the synthesis of a nucleic acid such as a DNA or an RNA. In some embodiments, the reaction mixture comprises a heat-stable enzyme that recognizes and removes U bases from DNA, e.g., a heat-stable uracil-DNA-N-glycosylase, that is actively removing U bases from DNA.

Embodiments of the technology related to reaction mixtures include a reaction mixture comprising a heat-stable enzyme that recognizes and removes U bases from DNA, e.g., a heat-stable uracil-DNA-N-glycosylase, in an amount of 0.1 unit, in an amount of 1.0 unit to 2.0 units, or in amounts of more than 2.0 units (e.g., 2.5 units, 3.0 units, 4.0 units, 5.0 units, 10 units, 20 units, or more). Related method embodiments comprise use of a heat-stable enzyme that recognizes and removes U bases from DNA, e.g., a heat-stable uracil-DNA-N-glycosylase, in an amount of in an amount of 0.1 unit, in an amount of 1.0 unit to 2.0 units, or in amounts of more than 2.0 units (e.g., 2.5 units, 3.0 units, 4.0 units, 5.0 units, 10 units, 20 units, or more).

In addition, embodiments of the technology related to reaction mixtures (and methods relating to use of reaction mixtures) include a reaction mixture comprising a heat-stable enzyme that recognizes and removes U bases from DNA, e.g., a heat-stable uracil-DNA-N-glycosylase, in an amount or at a concentration that is sufficient to remove U bases from DNA and minimize and/or prevent the proliferation of mutations (e.g., resulting from deamination, e.g., heat-induced deamination, of cytosines), e.g., in an amplification reaction. In addition, embodiments of the technology related to reaction mixtures include a reaction mixture comprising a heat-stable enzyme that recognizes and removes U bases from DNA, e.g., a heat-stable uracil-DNA-N-glycosylase, in an amount or at a concentration that is sufficient to minimize or eliminate errors in a DNA sequence due to deamination of cytosine residues. Further embodiments include reaction mixtures comprising a heat-stable enzyme that recognizes and removes U bases from DNA, e.g., a heat-stable uracil-DNA-N-glycosylase, during and after a period of heat-activation of a heat-activated polymerase at a rate that is at least 30%, at least 40%, at least 50%, at least 60%, at least 70%, at least 80%, at least 90%, or at least equal to or unchanged relative to the rate at which the heat-stable enzyme that recognizes and removes U bases from DNA, e.g., a heat-stable uracil-DNA-N-glycosylase, removes U bases prior to the period of heat-activation of the heat-activated polymerase. For example, embodiments include a reaction mixture comprising a heat-stable enzyme that recognizes and removes U bases from DNA, e.g., a heat-stable uracil-DNA-N-glycosylase, that has an activity during and after the heat-activation of a heat-activated polymerase that is at least 30%, at least 40%, at least 50%, at least 60%, at least 70%, at least 80%, at least 90%, or at least equal to or unchanged relative to the activity of the heat-stable enzyme that recognizes and removes U bases from DNA, e.g., a heat-stable uracil-DNA-N-glycosylase, prior to the heat-activation of a heat-activated polymerase.

Related embodiments comprise adding a heat-stable enzyme that recognizes and removes U bases from DNA to a composition that did not previously comprise such an enzyme. Additional embodiments relate to adding at least 0.1 unit of a heat-stable enzyme that recognizes and removes U bases from DNA to a composition that comprises less than 0.1 unit of such an enzyme, e.g., that comprised less than 0.1 unit prior to the addition provided by the present technology to provide a reaction mixture or composition according to the present technology.

Some related method embodiments comprise comparing the products of reactions (e.g., amplification reactions) comprising a heat-stable enzyme that recognizes and removes U bases from DNA, e.g., a heat-stable uracil-DNA-N-glycosylase, to reactions not comprising a heat-stable enzyme that recognizes and removes U bases from DNA, e.g., a heat-stable uracil-DNA-N-glycosylase. For example, these or similar comparisons are made to verify that the heat-stable enzyme that recognizes and removes U bases from DNA is active and or performed as expected in an amplification reaction, e.g., the products of a reaction mixture comprising a heat-stable enzyme that recognizes and removes U bases from DNA comprise fewer mutations resulting from the deamination of cytosines than the products of a reaction mixture not comprising a heat-stable enzyme that recognizes and removes U bases from DNA.

Accordingly, disclosed herein is technology relating to a method for detecting a target nucleic acid comprising a target sequence, the method comprising providing a sample comprising the target nucleic acid; adding at least 0.1 to 1 unit of a uracil-DNA glycosylase and a portion of the sample to a reaction mixture; exposing the uracil-DNA glycosylase to conditions in which the uracil-DNA glycosylase excises a uracil base from a damaged nucleic acid, if present; thermocycling the reaction mixture to produce an amplicon comprising the target sequence; and detecting the amplicon comprising the target sequence. In some embodiments, uracil-DNA glycosylase is a thermostable uracil-DNA glycosylase. Moreover, embodiments are disclosed wherein the reaction mixture comprises a polymerase and the method further comprises exposing the reaction mixture to a temperature that activates the polymerase. The technology relates to the active addition of a uracil-DNA glycosylase to a reaction mixture, e.g., to eliminate or minimize sequence errors detected in a nucleic acid detection assay, e.g., that result from heat-induced deamination of cytosine, such as in a PCR amplification to detect a SNP. As such, method embodiments are disclosed wherein an amount or concentration of the uracil-DNA glycosylase is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase. As such, in some embodiments, the damaged nucleic acid is not amplified and/or is not detected; in some embodiments, the damaged nucleic acid is amplified less than the target nucleic acid; in some embodiments, the damaged nucleic acid is detected at a later cycle than the target nucleic acid.

The technology is not limited in the detection method used. For example, in some embodiments, the detecting comprises using a labeled probe. In some embodiments, the detecting comprises sequencing; in some embodiments, the detecting comprises use of mass spectrometry; in some embodiments, the detecting comprises determining base composition. In some embodiments, the detection comprises determining a restriction pattern. In some embodiments, the detection comprises use of a flap endonuclease and one or more allele-specific probes (e.g., in an Invader assay; see, e.g., Olivier (2005) "The Invader assay for SNP genotyping" Mutat. Res. 573: 103-10). And, in some embodiments, the detecting comprises a separation technique such as chromatography, gel electrophoresis, and the like.

In some embodiments the technology relates to heat-damaged DNA, e.g., that results from a heat incubation of a heat-activated polymerase. As such, in some embodiments of the technology, the polymerase is a heat-activated polymerase. In some embodiments, a damaged nucleic acid is present and comprises a uracil base; in some embodiments, a damaged nucleic acid is present and comprises a deaminated cytosine.

In some embodiments, the target sequence is a single nucleotide polymorphism, e.g., in some embodiments, the target sequence comprises a cytosine or a guanine.

In some embodiments, the technology relates to a method for detecting a target nucleic acid comprising a target sequence (e.g., a SNP and/or comprising a guanine and/or a cytosine), the method comprising providing a sample comprising the target nucleic acid; adding at least 0.1 to 1.0 unit of an enzyme that removes uracil from DNA (e.g., a uracil-DNA glycosylase, e.g., a thermostable uracil-DNA glycosylase e.g., in an amount or concentration that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase) and a portion of the sample to a reaction mixture; exposing the uracil-DNA glycosylase to conditions in which the uracil-DNA glycosylase excises a uracil base from a damaged nucleic acid (e.g., comprising a uracil base, e.g., a deaminated cytosine), if present; thermocycling the reaction mixture to produce an amplicon comprising the target sequence; and contacting the amplicon with a nucleic acid probe (e.g., a labeled probe) specific for the target sequence, wherein the target nucleic acid is detected if the probe hybridizes to the target sequence; and/or the damaged nucleic acid is not amplified and/or is not detected; and/or the damaged nucleic acid is amplified less than the target nucleic acid.

In some embodiments, the technology relates to a method for detecting a target nucleic acid comprising a target sequence, the method comprising providing a sample comprising the target nucleic acid; adding at least 0.1 to 1.0 unit of a uracil-DNA glycosylase (e.g., a thermostable uracil-DNA glycosylase) and a portion of the sample to a reaction mixture comprising a polymerase (e.g., a heat-activated polymerase); exposing the uracil-DNA glycosylase to conditions in which the uracil-DNA glycosylase excises a uracil base from a damaged nucleic acid, if present; exposing the reaction mixture to a temperature that activates the polymerase; thermocycling the reaction mixture to produce an amplicon comprising the target sequence; and contacting the amplicon with a nucleic acid probe specific for the target sequence, wherein the target nucleic acid is detected if the probe hybridizes to the target sequence.

In some embodiments, the technology relates to a method for detecting a target nucleic acid comprising a target sequence (e.g., a SNP and/or comprising a guanine and/or a cytosine), the method comprising providing a sample comprising the target nucleic acid; adding at least 0.1 to 1.0 unit of an enzyme that removes uracil from DNA and cleaves DNA at abasic sites (e.g., a uracil-DNA glycosylase, e.g., a thermostable uracil-DNA glycosylase e.g., in an amount or concentration that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase) and a portion of the sample to a reaction mixture; exposing the uracil-DNA glycosylase to conditions in which the uracil-DNA glycosylase excises a uracil base from a damaged nucleic acid (e.g., comprising a uracil base, e.g., a deaminated cytosine), if present; thermocycling the reaction mixture to produce an amplicon comprising the target sequence; and contacting the amplicon with a nucleic acid probe (e.g., a labeled probe) specific for the target sequence, wherein the target nucleic acid is detected if the probe hybridizes to the target sequence; and/or the damaged nucleic acid is not amplified and/or is not detected; and/or the damaged nucleic acid is amplified less than the target nucleic acid.

In some embodiments, the technology relates to a method for detecting a target nucleic acid comprising a target sequence (e.g., a SNP and/or comprising a guanine and/or a cytosine), the method comprising providing a sample comprising the target nucleic acid; adding at least 0.1 to 1.0 unit of an enzyme that removes uracil from DNA (e.g., a uracil-DNA glycosylase, e.g., a thermostable uracil-DNA glycosylase e.g., in an amount or concentration that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase), an enzyme that cleaves DNA at an abasic site (e.g., an endonuclease), and a portion of the sample to a reaction mixture; exposing the uracil-DNA glycosylase to conditions in which the uracil-DNA glycosylase excises a uracil base from a damaged nucleic acid (e.g., comprising a uracil base, e.g., a deaminated cytosine), if present; thermocycling the reaction mixture to produce an amplicon comprising the target sequence; and contacting the amplicon with a nucleic acid probe (e.g., a labeled probe) specific for the target sequence, wherein the target nucleic acid is detected if the probe hybridizes to the target sequence; and/or the damaged nucleic acid is not amplified and/or is not detected; and/or the damaged nucleic acid is amplified less than the target nucleic acid.

The technology relates to minimizing sequence errors in amplicons as detected by a sequencing reaction. Accordingly, some embodiments include a method for detecting a target nucleic acid comprising a target sequence, the method comprising providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and sequencing the amplicon to determine a nucleic acid sequence of the amplicon, wherein the target nucleic acid is detected when the nucleic acid sequence of the amplicon comprises the target sequence.

The technology relates to minimizing sequence errors in amplicons as detected by a mass spectrometry technique. Accordingly, some embodiments include a method for detecting a target nucleic acid comprising a target sequence, the method comprising: providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and querying the amplicon by mass spectrometry to determine a chemical composition of the amplicon, wherein the target nucleic acid is detected when the chemical composition of the amplicon matches a chemical composition of the target sequence.

The technology relates to minimizing sequence errors in amplicons as detected by restriction analysis (e.g., use of a restriction enzyme to produce a restriction pattern such as in RFLP analysis). Accordingly, some embodiments include a method for detecting a target nucleic acid comprising a target sequence, the method comprising providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and contacting the amplicon with a restriction endonuclease to produce a restriction pattern, wherein the target nucleic acid is detected when the restriction pattern of the amplicon matches a restriction pattern of the target sequence.

The technology relates to minimizing sequence errors in amplicons as detected by an Invader assay. Accordingly, some embodiments include a method for detecting a target nucleic acid comprising a target sequence, the method comprising providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and contacting the amplicon with a flap endonuclease, wherein the target nucleic acid is detected when a flap endonuclease cleavage product is detected.

The technology relates to minimizing sequence errors in amplicons as detected by a primer extension assay. Accordingly, some embodiments include a method for detecting a target nucleic acid comprising a target sequence, the method comprising providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and contacting the amplicon with a primer for a primer extension assay, a nucleotide, and a polymerase, wherein the target nucleic acid is detected when the polymerase adds the nucleotide to the primer.

The technology relates to minimizing sequence errors in amplicons as detected by a ligation detection reaction. Accordingly, some embodiments include a method for detecting a target nucleic acid comprising a target sequence, the method comprising providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and contacting the amplicon with a first oligonucleotide, a second oligonucleotide, and a ligase, wherein the target nucleic acid is detected when the ligase ligates the first and second oligonucleotides.

The technology relates to minimizing sequence errors in amplicons as detected by a physical property of the amplicon (e.g., determining a melting temperature and/or melting profile, e.g., by high-resolution melting curve analysis, by single-strand conformation polymorphism (SSCP) analysis, by high resolution and/or high-performance liquid chromatography (HPLC) (e.g., denaturing HPLC), by electrophoresis (e.g., gel electrophoresis, e.g., temperature gradient gel electrophoresis), etc. Accordingly, some embodiments include a method for detecting a target nucleic acid comprising a target sequence, the method comprising providing a sample comprising the target nucleic acid; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; thermocycling the reaction mixture to produce an amplicon comprising the target sequence; and determining a physical property of the amplicon, wherein the target nucleic acid is detected when the physical property of the amplicon matches the physical property of the target sequence.

In some embodiments, the technology provides for eliminating, reducing, and/or minimizing sequence errors in a nucleic acid, e.g., an amplicon that is produced by a PCR. Sequence errors in an amplicon can be quantified in several ways. For instance, the sequence of the amplicon (e.g., a target sequence of the amplicon) can be aligned with or otherwise compared to a known sequence of the target nucleic acid (e.g., by comparison to a database or by other bioinformatic techniques) to determine a number of mismatches between the amplicon sequence and the known sequence. Determining mismatches in this way for amplicons produced according to the technology (e.g., in the presence of an enzyme that removes uracil from a nucleic acid) and for amplicons produced by conventional methods (e.g., without an enzyme that removes uracil from a nucleic acid) yields a quantitative and/or qualitative measurement of eliminating, reducing, and/or minimizing sequence errors in a nucleic acid by the technology.

Accordingly, in some embodiments are included a method of amplification for minimizing sequence errors in an amplicon comprising a target sequence, the method comprising providing a sample comprising a target nucleic acid comprising the target sequence; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; and thermocycling the reaction mixture to produce an amplicon comprising the target sequence, wherein the amplicon comprises fewer sequence errors resulting from the deamination of cytosine relative to the amplicon produced in the absence of at least 0.1 to 1.0 unit of an enzyme that removes uracil from DNA.

Some embodiments include a method of amplification for minimizing sequence errors in an amplicon comprising a target sequence, the method comprising providing a sample comprising a target nucleic acid comprising the target sequence; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present; and thermocycling the reaction mixture to produce an amplicon comprising the target sequence, wherein a first number of mismatches in the target sequence of the amplicon determined by alignment or comparison to the target sequence of the target nucleic acid in the sample is less than a second number of mismatches in the target sequence of an amplicon produced in the absence of the enzyme that removes uracil from DNA determined by alignment or comparison to the target sequence of the target nucleic acid in the sample.

Additional embodiments provide for treating a nucleic acid prior to amplification to remove any uracil bases that are present in the nucleic acid sample before amplification (e.g., as a result of other methods and/or handling of the sample). Accordingly, disclosed herein are embodiments of a method of amplification for minimizing sequence errors in an amplicon comprising a target sequence, the method comprising providing a sample comprising a target nucleic acid comprising the target sequence; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present, prior to thermocycling the reaction mixture to produce an amplicon comprising the target sequence, wherein the amplicon comprises fewer sequence errors resulting from the deamination of cytosine relative to the amplicon produced in the absence of at least 0.1 to 1.0 unit of an enzyme that removes uracil from DNA.

Some embodiments include a method of amplification for minimizing sequence errors in an amplicon comprising a target sequence, the method comprising providing a sample comprising a target nucleic acid comprising the target sequence; adding at least 0.1 to 1.0 unit of an enzyme that removes uracil from DNA and a portion of the sample to a reaction mixture; exposing the enzyme to conditions in which the enzyme excises a uracil base from a damaged nucleic acid, if present, prior to thermocycling the reaction mixture to produce an amplicon comprising the target sequence, wherein a first number of mismatches in the target sequence of the amplicon determined by alignment or comparison to the target sequence of the target nucleic acid in the sample is less than a second number of mismatches in the target sequence of an amplicon produced in the absence of the enzyme that removes uracil from DNA determined by alignment or comparison to the target sequence of the target nucleic acid in the sample.

Some embodiments include a method for detecting a target nucleic acid comprising a target sequence, the method comprising providing a sample comprising the target nucleic acid; adding at least 0.1 to 1.0 unit of an enzyme that removes uracil from DNA and also cleaves DNA at an abasic site and a portion of the sample to a reaction mixture; exposing the enzyme to conditions in which the enzyme excises a uracil base from a damaged nucleic acid and cleaves the damaged nucleic acid, if present; thermocycling the reaction mixture to produce an amplicon comprising the target sequence; and detecting the amplicon comprising the target sequence.

Some embodiments include a method for detecting a target nucleic acid comprising a target sequence, the method comprising providing a sample comprising the target nucleic acid; adding at least 0.1 to 1.0 unit of an enzyme that removes uracil from DNA, an enzyme that cleaves DNA at an abasic site, and a portion of the sample to a reaction mixture; exposing the enzyme to conditions in which the enzyme that removes uracil from DNA excises a uracil base from a damaged nucleic acid and the enzyme that cleaves DNA at an abasic site cleaves the damaged nucleic acid, if present; thermocycling the reaction mixture to produce an amplicon comprising the target sequence; and detecting the amplicon comprising the target sequence.

In other embodiments are disclosed a composition comprising a target nucleic acid comprising a target sequence, a polymerase, a uracil-DNA glycosylase, and a damaged nucleic acid comprising a uracil base. In some embodiments, the uracil-DNA glycosylase is a thermostable uracil-DNA glycosylase. In some embodiments, the composition further comprises a probe specific for the target sequence. Some particular embodiments disclose that the polymerase is a heat-activated polymerase. In certain embodiments, an amplicon results from an amplification reaction; accordingly in some embodiments the compositions of technology further comprise an amplicon comprising the target sequence. Embodiments relate to detecting SNPs; as such, in some embodiments the target sequence comprises a single nucleotide polymorphism. And, in some embodiments, the target sequence comprises a cytosine or a guanine.

In some embodiments are disclosed a composition comprising a target nucleic acid comprising a target sequence (e.g., comprising a SNP, e.g., comprising a cytosine and/or a guanine), a polymerase (e.g., a heat-activated polymerase), a uracil-DNA glycosylase (e.g., a thermostable uracil-DNA glycosylase), e.g., at least 0.1 to 1.0 units of a uracil-DNA glycosylase, a damaged nucleic acid comprising a uracil base, a probe (e.g., a labeled probe) specific for the target sequence, and an amplicon comprising the target sequence (e.g., comprising a SNP, e.g., comprising a cytosine and/or a guanine).

In some embodiments are disclosed a composition comprising a target nucleic acid comprising a target sequence (e.g., comprising a SNP, e.g., comprising a cytosine and/or a guanine), a polymerase (e.g., a heat-activated polymerase), a uracil-DNA glycosylase (e.g., a thermostable uracil-DNA glycosylase), e.g., an amount or concentration of the uracil-DNA glycosylase that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase, a damaged nucleic acid comprising a uracil base, a probe (e.g., a labeled probe) specific for the target sequence, and an amplicon comprising the target sequence (e.g., comprising a SNP, e.g., comprising a cytosine and/or a guanine).

In some embodiments are disclosed composition comprising a target nucleic acid comprising a target sequence (e.g., comprising a SNP, e.g., comprising a cytosine and/or a guanine), a polymerase (e.g., a heat-activated polymerase), a uracil-DNA glycosylase (e.g., a thermostable uracil-DNA glycosylase), e.g., an amount or concentration of the uracil-DNA glycosylase that is sufficient to remove uracil from the damaged nucleic acid during and/or after a period of heat-activation of the heat-activated polymerase, a damaged nucleic acid comprising a uracil base, a probe (e.g., a labeled probe) specific for the target sequence, and an amplicon comprising the target sequence (e.g., comprising a SNP, e.g., comprising a cytosine and/or a guanine).

The technology incudes embodiments of a composition comprising at least 0.1 to 1.0 units of the uracil-DNA glycosylase. The technology includes embodiments of a composition comprising an amount or concentration of the uracil-DNA glycosylase that is sufficient to remove uracil from DNA during and/or after a period of heating (e.g., during the heat-activation of a heat-activated polymerase) at a rate that is at least 30% of a rate at which the uracil-DNA glycosylase removes bases prior to the period of heating. The technology includes embodiments of a composition comprising an amount or concentration of the uracil-DNA glycosylase that is sufficient to remove an amount of uracil from DNA during and/or after a period of heating (e.g., during the heat-activation of a heat-activated polymerase) that is an amount at least 30% of the amount of uracil bases that the uracil-DNA glycosylase removes prior to the period of heating. As such, the technology includes embodiments of a composition comprising an amount or concentration of the uracil-DNA glycosylase that is sufficient to remove uracil from the damaged nucleic acid during and/or after a period of heat-activation of the heat-activated polymerase.

Kit embodiments are encompassed by the technology. For example, provided herein are embodiments of a kit for detecting a nucleic acid, wherein the kit comprises a first vessel comprising a heat-activated polymerase and a second vessel comprising a thermostable uracil-DNA glycosylase. In some embodiments, the kit comprises a vessel comprising a heat-activated polymerase and a thermostable uracil-DNA glycosylase. Some embodiments provide a kit that further comprises a control nucleic acid, e.g., as described herein. Some embodiments provide a kit for detecting a nucleic acid, the kit comprising a first vessel comprising a heat-activated polymerase; a second vessel comprising a thermostable uracil-DNA glycosylase; and the kit further comprising a control nucleic acid. Some embodiments provide a kit for detecting a nucleic acid, the kit comprising a vessel comprising a heat-activated polymerase and a thermostable uracil-DNA glycosylase, and the kit further comprising a control nucleic acid.

Embodiments of the technology include a method for detecting a target nucleic acid comprising a target sequence, the method comprising providing a sample comprising the target nucleic acid; adding at least 0.1 to 1.0 unit of a uracil-DNA glycosylase and a portion of the sample to a reaction mixture; exposing the uracil-DNA glycosylase to conditions in which the uracil-DNA glycosylase excises a uracil base from a damaged nucleic acid, if present; thermocycling the reaction mixture to produce an amplicon comprising the target sequence; and detecting the amplicon comprising the target sequence.

Embodiments of the technology include a method for detecting a target nucleic acid comprising a target sequence, the method comprising providing a sample comprising the target nucleic acid; adding at least 0.1 to 1.0 unit of a thermostable uracil-DNA glycosylase and a portion of the sample to a reaction mixture; exposing the thermostable uracil-DNA glycosylase to conditions in which the thermostable uracil-DNA glycosylase excises a uracil base from a damaged nucleic acid, if present; thermocycling the reaction mixture to produce an amplicon comprising the target sequence; and detecting the amplicon comprising the target sequence.

Embodiments of the technology include a method for detecting a target nucleic acid comprising a target sequence (e.g., a SNP, e.g., comprising a cytosine or guanine), the method comprising providing a sample comprising the target nucleic acid; adding at least 0.1 to 1.0 unit of a uracil-DNA glycosylase and a portion of the sample to a reaction mixture (e.g., comprising a polymerase; e.g., a heat-activated polymerase; e.g., a heat-activated, heat-stable polymerase); exposing the uracil-DNA glycosylase to conditions in which the uracil-DNA glycosylase excises a uracil base from a damaged nucleic acid, if present (e.g., a heat-damaged DNA, e.g., a DNA comprising a deaminated cytosine that results from, e.g., heat-induced deamination, e.g., as occurs during PCR, e.g., during heat incubation of a heat-activated polymerase); thermocycling the reaction mixture to produce an amplicon comprising the target sequence; and detecting the amplicon comprising the target sequence (e.g., detecting the amplicon using a labeled probe; sequencing the amplicon; acquiring mass spectrometry data from the amplicon; detecting the amplicon by electrophoresis; and/or determining a base composition of the amplicon).

Embodiments of the technology include a method for detecting a target nucleic acid comprising a target sequence (e.g., a SNP, e.g., comprising a cytosine or guanine), the method comprising providing a sample comprising the target nucleic acid; adding at least 0.1 to 1.0 unit of a uracil-DNA glycosylase and a portion of the sample to a reaction mixture (e.g., comprising a polymerase; e.g., a heat-activated polymerase; e.g., a heat-activated, heat-stable polymerase); exposing the reaction mixture to a temperature that activates the heat-activated polymerase; exposing the uracil-DNA glycosylase to conditions in which the uracil-DNA glycosylase excises a uracil base from a damaged nucleic acid, if present (e.g., a heat-damaged DNA, e.g., a DNA comprising a deaminated cytosine that results from, e.g., heat-induced deamination, e.g., as occurs during PCR, e.g., during heat incubation of a heat-activated polymerase); thermocycling the reaction mixture to produce an amplicon comprising the target sequence; and detecting the amplicon comprising the target sequence (e.g., detecting the amplicon using a labeled probe; sequencing the amplicon; acquiring mass spectrometry data from the amplicon; and/or determining a base composition of the amplicon).

Embodiments of the technology include a method for detecting a target nucleic acid comprising a target sequence (e.g., a SNP, e.g., comprising a cytosine or guanine), the method comprising providing a sample comprising the target nucleic acid; adding an amount or concentration of a uracil-DNA glycosylase that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase and a portion of the sample to a reaction mixture (e.g., comprising a polymerase; e.g., a heat-activated polymerase; e.g., a heat-activated, heat-stable polymerase); exposing the reaction mixture to a temperature that activates the heat-activated polymerase; exposing the uracil-DNA glycosylase to conditions in which the uracil-DNA glycosylase excises a uracil base from a damaged nucleic acid, if present (e.g., a heat-damaged DNA, e.g., a DNA comprising a deaminated cytosine that results from, e.g., heat-induced deamination, e.g., as occurs during PCR, e.g., during heat incubation of a heat-activated polymerase); thermocycling the reaction mixture to produce an amplicon comprising the target sequence; and detecting the amplicon comprising the target sequence (e.g., detecting the amplicon using a labeled probe; sequencing the amplicon; acquiring mass spectrometry data from the amplicon; and/or determining a base composition of the amplicon).

Embodiments of the technology include a method for detecting a target nucleic acid comprising a target sequence (e.g., a SNP, e.g., comprising a cytosine or guanine), the method comprising providing a sample comprising the target nucleic acid; adding an amount or concentration of a uracil-DNA glycosylase that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase and a portion of the sample to a reaction mixture (e.g., comprising a polymerase; e.g., a heat-activated polymerase; e.g., a heat-activated, heat-stable polymerase); exposing the reaction mixture to a temperature that activates the heat-activated polymerase; exposing the uracil-DNA glycosylase to conditions in which the uracil-DNA glycosylase excises a uracil base from a damaged nucleic acid, if present (e.g., a heat-damaged DNA, e.g., a DNA comprising a deaminated cytosine that results from, e.g., heat-induced deamination, e.g., as occurs during PCR, e.g., during heat incubation of a heat-activated polymerase); thermocycling the reaction mixture to produce an amplicon comprising the target sequence; and detecting the amplicon comprising the target sequence (e.g., detecting the amplicon using a labeled probe; sequencing the amplicon; acquiring mass spectrometry data from the amplicon; and/or determining a base composition of the amplicon), wherein the damaged nucleic acid is not amplified and/or is not detected; and/or the damaged nucleic acid is amplified less than the target nucleic acid.

Embodiments of the technology include a composition comprising a target nucleic acid comprising a target sequence, a polymerase, a thermostable uracil-DNA glycosylase, and a damaged nucleic acid comprising a uracil base. Embodiments of the technology include a composition comprising a target nucleic acid comprising a target sequence, a polymerase, a thermostable uracil-DNA glycosylase, a probe specific for the target sequence, and a damaged nucleic acid comprising a uracil base. Embodiments of the technology include a composition comprising a target nucleic acid comprising a target sequence, a polymerase, a uracil-DNA glycosylase, a probe specific for the target sequence, and a damaged nucleic acid comprising a uracil base.

Embodiments of the technology include a composition comprising a target nucleic acid comprising a target sequence, a polymerase (e.g., a heat-activated polymerase), a thermostable uracil-DNA glycosylase, and an amplicon comprising the target sequence.

Some embodiments include a composition comprising an enzyme that cleaves a nucleic acid at an abasic site, e.g., a nuclease, e.g., a heat-stable nuclease.

Embodiments of the technology include a composition comprising a target nucleic acid comprising a target sequence (e.g., comprising a SNP, e.g., comprising a cytosine or a guanine), a polymerase (e.g., a heat-activated and/or heat-stable polymerase), at least 0.1 to 1.0 unit (e.g., at least 0.1 unit, at least 1.0 unit, at least 2.0 units, at least 2.5 units, at least 3.0 units, at least 4.0 units, at least 5.0 units, at least 10 units, at least 20 units or more) of a thermostable uracil-DNA glycosylase, and a damaged nucleic acid comprising a uracil base.

Embodiments of the technology include a composition comprising a target nucleic acid comprising a target sequence (e.g., comprising a SNP, e.g., comprising a cytosine or a guanine), a polymerase (e.g., a heat-activated and/or heat-stable polymerase), at least an amount or concentration of a uracil-DNA glycosylase that is sufficient to remove uracil from a DNA during and/or after a period of heat-activation of a heat-activated polymerase at a rate that is at least 30% of a rate at which the uracil-DNA glycosylase removes bases prior to the period of heat-activation of the heat-activated polymerase of a thermostable uracil-DNA glycosylase, and a damaged nucleic acid comprising a uracil base.

Embodiments of the technology include a composition comprising a target nucleic acid comprising a target sequence (e.g., comprising a SNP, e.g., comprising a cytosine or a guanine), a polymerase (e.g., a heat-activated and/or heat-stable polymerase), at least an amount or concentration of a uracil-DNA glycosylase that is sufficient to remove uracil from a damaged nucleic acid during and/or after a period of heat-activation of the heat-activated polymerase., and a damaged nucleic acid comprising a uracil base.

Kit embodiments are provided that comprise a first vessel comprising a heat-activated polymerase, a second vessel comprising a thermostable uracil-DNA glycosylase, and a control nucleic acid (e.g., a positive control and/or a negative control). Kit embodiments are provided that comprise a vessel comprising a heat-activated polymerase and a thermostable uracil-DNA glycosylase; and a control nucleic acid (e.g., a positive control and/or a negative control).

Additional embodiments will be apparent to persons skilled in the relevant art based on the teachings contained herein. For example, while the technology is described in relation to heat-stable enzymes (e.g., UDG) used to minimize errors due to heat-induced modifications of DNA (e.g., deamination of C bases to form U bases), the technology also relates to other enzymes stable in a variety of conditions in which similar modifications of DNA take place. For example, the technology contemplates the use of enzymes stable at high or low pH where similar deamination (e.g., of C bases) occurs. For instance, some polymerases are activated by a change in pH rather than heat activation (though, in some embodiments, the change in pH is effected by a change in temperature), and the technology encompasses a pH-stable enzyme for removing uracil from a nucleic acid (e.g., a uracil that occur as a result of a change in pH, e.g., as a result of pH-induced deamination of cytosine). The technology similarly contemplates enzymes that are stable in various milieux to counteract the effects of pressure, ionic strength, organic solvents and other chemicals, etc. on DNA bases.

Some embodiments provide for assessing genetic variation (e.g., by detecting one or more SNPs) by generating a sequence-specific signal, recording the sequence-specific signal, and analyzing the signal. In particular, some embodiments comprise processing raw data (e.g., quantitative or qualitative raw data) to identify SNPs, SNP frequencies, and/or tissue-specific expression patterns and/or expression levels of SNPs. See, e.g., Wang et al (2007) "SNP and mutation analysis" Adv. Exp. Med. Biol. 593: 105-16.

The technology also finds use in removing uracil from other nucleic acids, e.g., in naturally occurring nucleic acids, such as uracils introduced into nucleic acids during antibody diversification or class switching.

### BRIEF DESCRIPTION OF THE DRAWINGS

These and other features, aspects, and advantages of the present technology will become better understood with regard to the following drawings:

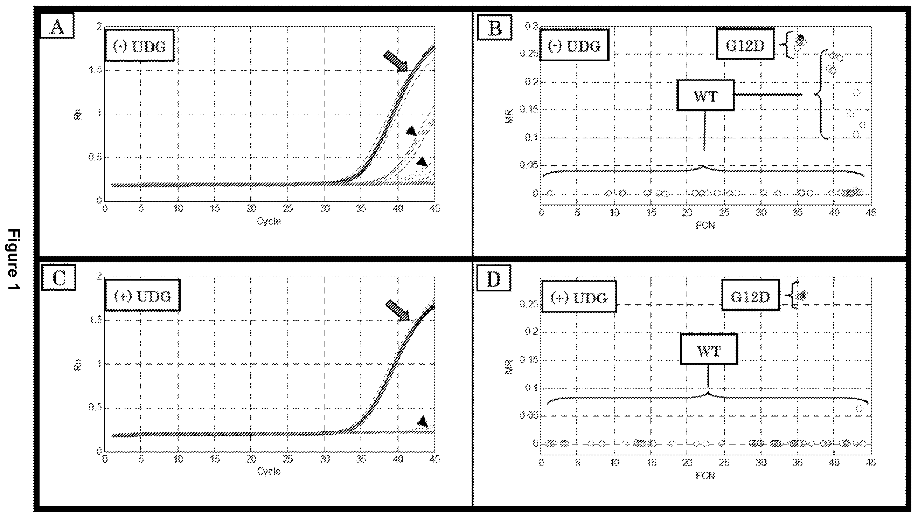

Figure 1A is a real-time PCR plot of G12D mutant KRAS detection in 42 replicates of 5 ng of human genomic DNA containing 100% wild-type KRAS sequence (arrow heads) and 6 replicates of 5 ng of human genomic DNA containing 1% G12D mutant KRAS sequence (arrow) performed in the absence of UDG. The x-axis depicts the PCR cycle number and the y-axis depicts relative fluorescence units. Figure 1B is a MaxRatio analysis of the experiment performed in 1A (see, e.g., Shain and Clements (2008), "A new method for robust quantitative and qualitative analysis of real-time PCR" Nucl. Acids Res. 36: e91). The x-axis represents
   cycle number (analogous to Ct) and the y-axis depicts the MaxRatio. Each replicate is individually plotted. Figure 1C is a real-time PCR plot of G12D mutant KRAS detection in 42 replicates of 5 ng of human genomic 24 ntaining 100% wild-type KRAS sequence (arrow head) and 6 replicates of 5 ng of human genomic DNA containing 1% G12D mutant KRAS sequence (arrow) performed in the presence of UDG. The x-axis depicts the PCR cycle number and the y-axis depicts relative fluorescence units. Figure 1D is a MaxRatio analysis of the experiment performed in 1C. The x-axis represents cycle number (analogous to Ct) and the y-axis depicts the MaxRatio. Each replicate is individually plotted.

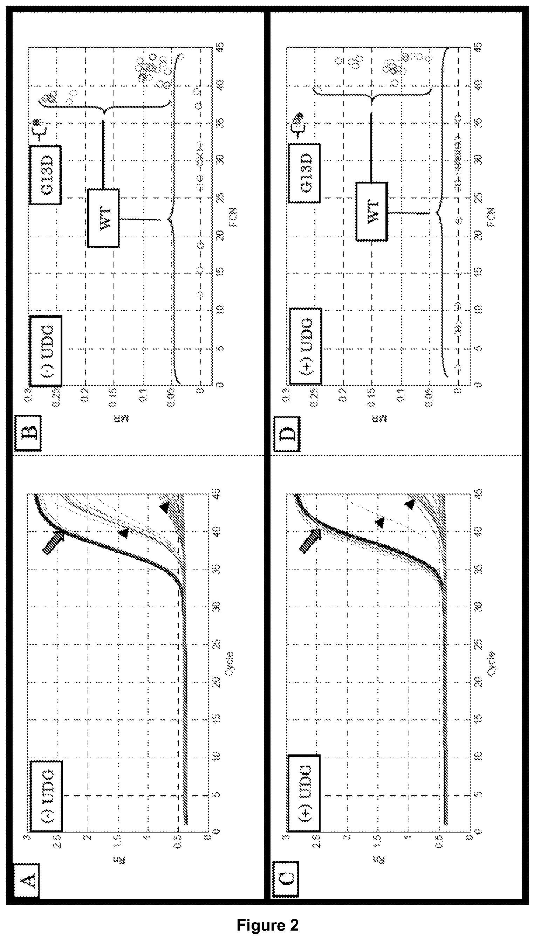

Figure 2A is a real-time PCR plot of G13D mutant KRAS detection in 42 replicates of 5 ng of human genomic DNA containing 100% wild-type KRAS sequence (arrow heads) and 6 replicates of 5 ng of human genomic DNA containing 1% G13D mutant KRAS sequence (arrow) performed in the absence of UDG. The x-axis depicts the PCR cycle number and the y-axis depicts relative fluorescence units. Figure 2B is a MaxRatio analysis of the experiment performed in 2A. The x-axis represents cycle number (analogous to Ct) and the y-axis depicts the MaxRatio. Each replicate is individually plotted. Figure 2C is a real-time PCR plot of G13D mutant KRAS detection in 42 replicates of 5 ng of human genomic DNA containing 100% wild-type KRAS sequence (arrow heads) and 6 replicates of 5 ng of human genomic DNA containing 1% G13D mutant KRAS sequence (arrow) performed in the presence of UDG. The x-axis depicts the PCR cycle number and the y-axis depicts relative fluorescence units. Figure 2D is a MaxRatio analysis of the experiment performed in 2C. The x-axis represents cycle number (analogous to Ct) and the y-axis depicts the MaxRatio. Each replicate is individually plotted.

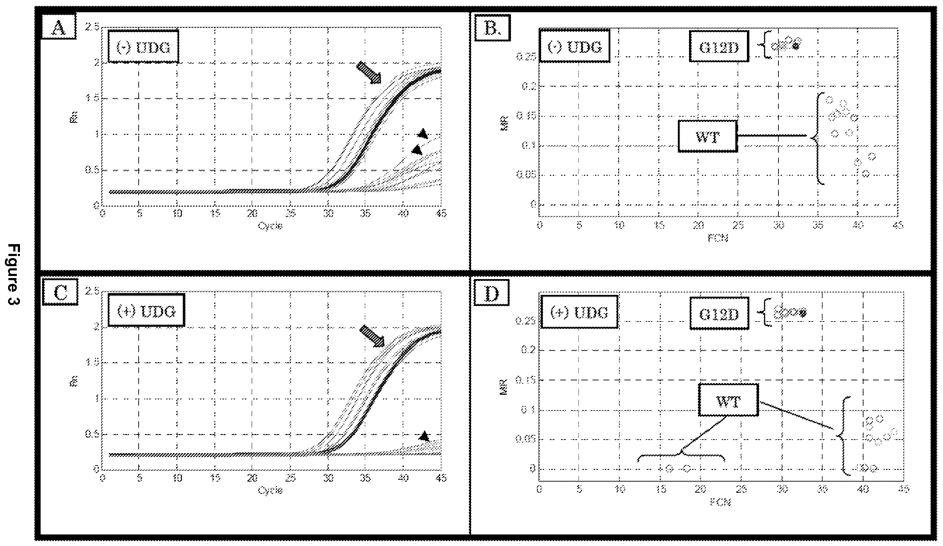

Figure 3A is a real-time PCR plot of G12D mutant KRAS detection in 12 replicates ranging from 50 ng to 400 ng of human genomic DNA containing 100% wild-type KRAS sequence (arrow heads) and 12 replicates ranging from 50 ng to 400 ng of human genomic DNA containing 1% G12D mutant KRAS sequence (arrow) performed in the absence of UDG. The x-axis depicts the PCR cycle number and the y-axis depicts relative fluorescence units. Figure 3B is a MaxRatio analysis of the experiment performed in 3A. The x-axis represents cycle number (analogous to Ct) and the y-axis depicts the MaxRatio. Each replicate is individually plotted. Figure 3C is a real-time PCR plot of G12D mutant KRAS detection in 12 replicates ranging from 50 ng to 400 ng of human genomic DNA containing 100% wild-type KRAS sequence (arrow head) and 12 replicates ranging from 50 ng to 400 ng of human genomic DNA containing 1% G12D mutant KRAS sequence (arrow) performed in the presence of UDG. The x-axis depicts the PCR cycle number and the y-axis depicts relative fluorescence units. Figure 3D is a MaxRatio analysis of the experiment performed in 3C. The x-axis represents cycle number (analogous to Ct) and the y-axis depicts the MaxRatio. Each replicate is individually plotted.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### DETAILED DESCRIPTION

Disclosed herein is technology relating to enzymatic modification of nucleic acids and particularly, but not exclusively, to methods and compositions relating to using uracil-DNA-N-glycosylase for minimizing or eliminating errors in a DNA sequence due to deamination of cytosine residues.

The section headings used herein are for organizational purposes only and are not to be construed as limiting the described subject matter in any way.

In this detailed description of the various embodiments, for purposes of explanation, numerous specific details are set forth to provide a thorough understanding of the embodiments disclosed. One skilled in the art will appreciate, however, that these various embodiments may be practiced with or without these specific details. In other instances, structures and devices are shown in block diagram form. Furthermore, one skilled in the art can readily appreciate that the specific sequences in which methods are presented and performed are illustrative and it is contemplated that the sequences can be varied.

### Summary of the invention

The present invention provides a composition comprising a target nucleic acid comprising: a) a target sequence, b) a heat-activated polymerase, c) at least one of: i) a heat stable enzyme that removes uracil from DNA and cleaves DNA at an abasic site; ii) a heat stable enzyme that removes uracil from DNA and an enzyme that cleaves DNA at an abasic site or iii) a heat stable uracil-DNA glycosylase, and d) a damaged nucleic acid comprising a uracil base.

The present invention also provides a kit for detecting a nucleic acid, the kit comprising:
a) a first vessel comprising a heat-activated polymerase; and a second vessel comprising a thermostable uracil-DNA glycosylase; or
b) a vessel comprising a heat-activated polymerase and a thermostable uracil-DNA glycosylase;
c) a first vessel comprising a heat-activated polymerase; and a second vessel comprising a heat stable enzyme that removes uracil from DNA;
d) a first vessel comprising a heat-activated polymerase; and a second vessel comprising a heat stable enzyme that removes uracil from DNA and that cleaves DNA at an abasic site;
e) a first vessel comprising a heat-activated polymerase; a second vessel comprising a heat stable enzyme that removes uracil from DNA; and a third vessel comprising an enzyme that cleaves DNA at an abasic site; or
f) a first vessel comprising a heat-activated polymerase; and a second vessel comprising a heat stable enzyme that removes uracil from DNA and an enzyme that cleaves DNA at an abasic site.

The present invention also provides a method for detecting a target nucleic acid comprising a target sequence, the method comprising:
a) providing a sample comprising the target nucleic acid;
b) adding at least one of: i) a heat stable enzyme that removes uracil from DNA and cleaves DNA at an abasic site; ii) a heat stable enzyme that removes uracil from DNA and an enzyme that cleaves DNA at an abasic site or iii) a heat stable uracil-DNA glycosylase;
c) excising a uracil base from a damaged nucleic acid, if present;
d) thermocycling the reaction mixture to produce an amplicon comprising the target sequence; and
e) detecting the amplicon comprising the target sequence wherein the damaged nucleic acid is not amplified, is not detected, and/or is amplified less than the target nucleic acid, if present.

### Definitions

To facilitate an understanding of the present technology, a number of terms and phrases are defined below. Additional definitions are set forth throughout the detailed description.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, the term "nucleic acid molecule" refers to any nucleic acid containing molecule, including but not limited to, DNA or RNA. The term encompasses sequences that include any of the known base analogs of DNA and RNA including, but not limited to, 4-acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, N6-methyladenine, 7-methylguanine, 5-methylaminomethyluracil, 5-methoxy-amino-methyl-2-thiouracil, beta- D mannosylqueosine, 5'-methoxycarbonylmethyluracil, 5-methoxyuracil, 2-methylthio- N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5- oxyacetic acid, pseudouracil, queosine, 2-thiocytosine, and 2,6-diaminopurine.

As used herein, a "damaged nucleic acid" includes, e.g., a DNA comprising a deaminated base (e.g., a deaminated cytosine) or a DNA comprising a uracil base.

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP). As is used herein, a nucleobase includes natural and modified residues, as described herein.

It is well known that DNA (deoxyribonucleic acid) is a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. It is also known that all of these 5 types of nucleotides specifically bind to one another in combinations called complementary base pairing. That is, adenine (A) pairs with thymine (T) (in the case of RNA, however, adenine (A) pairs with uracil (U)), and cytosine (C) pairs with guanine (G), so that each of these base pairs forms a double strand. In some instances, one or more nucleotides are referred to by a code as follows: R (G or A), Y (T/U or C), M (A or C), K (G or T/U), S (G or C), W (A or T/U), B (G or C or T/U), D (A or G or T/U), H (A or C or T/U), V (A or G or C), or N (A or G or C or T/U), gap (-).

The terms "protein" and "polypeptide" refer to compounds comprising amino acids joined via peptide bonds and are used interchangeably. Conventional one and three-letter amino acid codes are used herein as follows - Alanine: Ala, A; Arginine: Arg, R; Asparagine: Asn, N; Aspartate: Asp, D; Cysteine: Cys, C; Glutamate: Glu, E; Glutamine: Gln, Q; Glycine: Gly, G; Histidine: His, H; Isoleucine: Ile, I; Leucine: Leu, L; Lysine: Lys, K; Methionine: Met, M; Phenylalanine: Phe, F; Proline: Pro, P; Serine: Ser, S; Threonine: Thr, T; Tryptophan: Trp, W; Tyrosine: Tyr, Y; Valine: Val, V. As used herein, the codes Xaa and X refer to any amino acid.

An "oligonucleotide" refers to a nucleic acid that includes at least two nucleic acid monomer units (e.g., nucleotides), typically more than three monomer units, and more typically greater than ten monomer units. The exact size of an oligonucleotide generally depends on various factors, including the ultimate function or use of the oligonucleotide. To further illustrate, oligonucleotides are typically less than 200 residues long (e.g., between 15 and 100), however, as used herein, the term is also intended to encompass longer polynucleotide chains. Oligonucleotides are often referred to by their length. For example a 24 residue oligonucleotide is referred to as a "24-mer". Typically, the nucleoside monomers are linked by phosphodiester bonds or analogs thereof, including phosphorothioate, phosphorodithioate, phosphoroselenoate, phosphorodiselenoate, phosphoroanilothioate, phosphoranilidate, phosphoramidate, and the like, including associated counterions, e.g., H⁺, NH₄⁺, Na⁺, and the like, if such counterions are present. Further, oligonucleotides are typically single-stranded. Oligonucleotides are optionally prepared by any suitable method, including, but not limited to, isolation of an existing or natural sequence, DNA replication or amplification, reverse transcription, cloning and restriction digestion of appropriate sequences, or direct chemical synthesis by a method such as the phosphotriester method of Narang et al. (1979) Meth Enzymol. 68:90-99; the phosphodiester method of Brown et al. (1979) Meth Enzymol. 68:109-151; the diethylphosphoramidite method of Beaucage et al. (1981) Tetrahedron Lett. 22:1859-1862; the triester method of Matteucci et al. (1981) J Am Chem Soc 103:3185-3191; automated synthesis methods; or the solid support method of U.S. Pat. No. 4,458,066, entitled "PROCESS FOR PREPARING POLYNUCLEOTIDES," issued Jul. 3, 1984 to Caruthers et al., or other methods known to those skilled in the art.

A "sequence" of a biopolymer (e.g., a nucleic acid) refers to the order and identity of monomer units (e.g., nucleotides, etc.) in the biopolymer. The sequence (e.g., base sequence) of a nucleic acid is typically read in the 5' to 3' direction.

The term "gene" refers to a nucleic acid (e.g., DNA or RNA) sequence that comprises coding sequences necessary for the production of an RNA, or a polypeptide or its precursor (e.g., proinsulin). A functional polypeptide can be encoded by a full length coding sequence or by any portion of the coding sequence as long as the desired activity or functional properties (e.g., enzymatic activity, ligand binding, signal transduction, etc.) of the polypeptide are retained. The term "portion" when used in reference to a gene refers to fragments of that gene. The fragments may range in size from a few nucleotides to the entire gene sequence minus one nucleotide. Thus, "a nucleotide comprising at least a portion of a gene" may comprise fragments of the gene or the entire gene.

The term "gene" also encompasses the coding regions of a structural gene and includes sequences located adjacent to the coding region on both the 5' and 3' ends for a distance of about 1 kb on either end such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences. The term "gene" encompasses both cDNA and genomic forms of a gene. A genomic form or clone of a gene contains the coding region interrupted with non-coding sequences termed "introns" or "intervening regions" or "intervening sequences." Introns are segments of a gene which are transcribed into nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

In addition to containing introns, genomic forms of a gene may also include sequences located on both the 5' and 3' end of the sequences which are present on the RNA transcript. These sequences are referred to as "flanking" sequences or regions (these flanking sequences are located 5' or 3' to the non-translated sequences present on the mRNA transcript). The 5' flanking region may contain regulatory sequences such as promoters and enhancers which control or influence the transcription of the gene. The 3' flanking region may contain sequences which direct the termination of transcription, posttranscriptional cleavage and polyadenylation.

The term "nucleotide sequence of interest" or "nucleic acid sequence of interest" or "target" or "target nucleic acid" refers to any nucleotide sequence (e.g., RNA or DNA), the manipulation of which may be deemed desirable for any reason (e.g., treat disease, confer improved qualities, etc.), by one of ordinary skill in the art. Such nucleotide sequences include, but are not limited to, coding sequences of structural genes (e.g., reporter genes, selection marker genes, oncogenes, drug resistance genes, growth factors, etc.), and non-coding regulatory sequences which do not encode an mRNA or protein product (e.g., promoter sequence, polyadenylation sequence, termination sequence, enhancer sequence, etc.)

As used herein, the term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced (e.g., in the presence of nucleotides and an inducing agent such as a biocatalyst (e.g., a DNA polymerase or the like) and at a suitable temperature and pH). The primer is typically single stranded for maximum efficiency in amplification, but may alternatively be double stranded. If double stranded, the primer is generally first treated to separate its strands before being used to prepare extension products. In some embodiments, the primer is an oligodeoxyribonucleotide. The primer is sufficiently long to prime the synthesis of extension products in the presence of the inducing agent. The exact lengths of the primers will depend on many factors, including temperature, source of primer and the use of the method.

As used herein, the term "amplicon" refers to a nucleic acid generated using an amplification method as described herein. The amplicon is typically double stranded DNA; however, an amplicon may be RNA and/or a DNA:RNA hybrid. In some embodiments, the amplicon comprises DNA complementary to target RNA, DNA, or cDNA. In some embodiments, primer pairs are configured to generate amplicons from a target nucleic acid. In certain embodiments, after amplification of the target region using the primers the resultant amplicons having the primer sequences are used to generate signal that detects, identifies, or otherwise analyzes the nucleic acid from the tested sample.

The term "amplifying" or "amplification" in the context of nucleic acids refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes. The generation of multiple DNA copies from one or a few copies of a target or template DNA molecule during a polymerase chain reaction (PCR) or a ligase chain reaction (LCR) are forms of amplification. Amplification is not limited to the strict duplication of the starting molecule. For example, the generation of multiple cDNA molecules from a limited amount of RNA in a sample using reverse transcription (RT)-PCR is a form of amplification. Furthermore, the generation of multiple RNA molecules from a single DNA molecule during the process of transcription is also a form of amplification.

As used herein, the terms "complementary" or "complementarity" are used in reference to polynucleotides (e.g., a sequence of nucleotides) related by the base-pairing rules. For example, the sequence 5'-A-G-T-3' is complementary to the sequence 3'-T-C-A-5'. Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods that depend upon.

The term "wild-type" when made in reference to a gene refers to a gene that has the characteristics of a gene isolated from a naturally occurring source. The term "wild-type" when made in reference to a gene product (e.g., a polypeptide) refers to a gene product that has the characteristics of a gene product isolated from a naturally occurring source. The term "naturally-occurring" as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring. A wild-type gene is frequently that gene which is most frequently observed in a population and is thus arbitrarily designated the "normal" or "wild-type" form of the gene. In contrast, the term "modified" or "mutant" when made in reference to a gene or to a gene product refers, respectively, to a gene or to a gene product which displays modifications in sequence and/or functional properties (e.g., altered characteristics) when compared to the wild-type gene or gene product. It is noted that naturally-occurring mutants can be isolated; these are identified by the fact that they have altered characteristics when compared to the wild-type gene or gene product.

The term "allele" refers to different variations in a gene; the variations include but are not limited to variants and mutants, polymorphic loci and single nucleotide polymorphic (SNP) loci, frameshift and splice mutations. An allele may occur naturally in a population, or it might arise during the lifetime of any particular individual of the population.

Thus, the terms "variant" and "mutant" when used in reference to a nucleotide sequence refer to an nucleic acid sequence that differs by one or more nucleotides from another, usually related nucleotide acid sequence. A "variation" is a difference between two different nucleotide sequences; typically, one sequence is a reference sequence.

The terms "variant" and "mutant" when used in reference to a polypeptide refer to an amino acid sequence that differs by one or more amino acids from another (a "substitution" of one amino acid for another), usually related polypeptide.

The nomenclature used to describe variants of nucleic acids or proteins specifies the type of mutation and base or amino acid changes. For a nucleotide substitution (e.g., 76A>T), the number is the position of the nucleotide from the 5' end, the first letter represents the wild type nucleotide, and the second letter represents the nucleotide which replaced the wild type. In the given example, the adenine at the 76th position was replaced by a thymine. If it becomes necessary to differentiate between mutations in genomic DNA, mitochondrial DNA, complementary DNA (cDNA), and RNA, a simple convention is used. For example, if the 100th base of a nucleotide sequence is mutated from G to C, then it would be written as g.100G>C if the mutation occurred in genomic DNA, m.100G>C if the mutation occurred in mitochondrial DNA, c.100G>C if the mutation occurred in cDNA, or r.100g>c if the mutation occurred in RNA.

For amino acid substitution (e.g., D111E), the first letter is the one letter code of the wild type amino acid, the number is the position of the amino acid from the N-terminus, and the second letter is the one letter code of the amino acid present in the mutation. Nonsense mutations are represented with an X for the second amino acid (e.g. D111X). For amino acid deletions (e.g. ΔF508, F508del), the Greek letter Δ (delta) or the letters "del" indicate a deletion. The letter refers to the amino acid present in the wild type and the number is the position from the N terminus of the amino acid where it is present in the wild type. Intronic mutations are designated by the intron number or cDNA position and provide either a positive number starting from the G of the GT splice donor site or a negative number starting from the G of the AG splice acceptor site. g.3' +7G>C denotes the G to C substitution at nt +7 at the genomic DNA level. When the full-length genomic sequence is known, the mutation is best designated by the nucleotide number of the genomic reference sequence. See den Dunnen & Antonarakis, "Mutation nomenclature extensions and suggestions to describe complex mutations: a discussion". Human Mutation 15: 7-12 (2000); Ogino S, et al., "Standard Mutation Nomenclature in Molecular Diagnostics: Practical and Educational Challenges", J. Mol. Diagn. 9(1): 1-6 (February 2007).

As used herein, the one-letter codes for amino acids refer to standard IUB nomenclature as described in "IUPAC-IUB Nomenclature of Amino Acids and Peptides" published in Biochem. J., 1984, 219, 345-373; Eur. J. Biochem., 1984, 138, 9-37; 1985, 152, 1; Internat. J. Pept. Prot. Res., 1984, 24, following p 84; J. Biol. Chem., 1985, 260, 14-42; Pure Appl. Chem., 1984, 56, 595-624; Amino Acids and Peptides, 1985, 16, 387-410; and in Biochemical Nomenclature and Related Documents, 2nd edition, Portland Press, 1992, pp 39-67.

As used herein, an "abasic site" refers to a location in DNA that has neither a purine nor a pyrimidine base, either spontaneously or due to DNA damage. An abasic site is also known as an apurinic/apyrimidinic or an AP site. Abasic sites can be formed by spontaneous depurination, but also occur as intermediates in base excision repair. In this process, a DNA glycosylase (e.g., UDG) recognizes a damaged base and cleaves the N-glycosidic bond to release the base, leaving an AP site.

The term "detection assay" refers to an assay for detecting the presence or absence of a wild-type or variant nucleic acid sequence (e.g., mutation or polymorphism) in a given allele of a particular gene, or for detecting the presence or absence of a particular protein or the activity or effect of a particular protein or for detecting the presence or absence of a variant of a particular protein.

The term "detect", "detecting", or "detection" refers to an act of determining the existence or presence of one or more targets (e.g., an amplicon, a SNP, etc.) in a sample.

The term "sample" is used in its broadest sense. In one sense it can refer to an animal cell or tissue. In another sense, it is meant to include a specimen or culture obtained from any source, as well as biological and environmental samples. Biological samples may be obtained from plants or animals (including humans) and encompass fluids, solids, tissues, and gases. Environmental samples include environmental material such as surface matter, soil, water, and industrial samples. These examples are not to be construed as limiting the sample types applicable to the present invention.

As used herein, a "UDG" is a uracil-DNA glycosylase, also called a uracil-DNA N-glycosylase. Uracil-DNA N-glycosylase enzymes excise uracil from DNA by cleaving the N-glycosidic bond between the uracil base and the sugar backbone. This cleavage generates abasic sites that are blocked from replication by DNA polymerase or prevented from becoming a hybridization site. Double-stranded DNA and single-stranded DNA are substrates for uracil-DNA N-glycosylase. In some organisms, the gene encoding a uracil-DNA N-glycosylase is known as the"UNG" gene.

As used herein, the term "heat-stable" or "thermostable" as used in reference to an enzyme, such as a uracil-DNA N-glycosylase, indicates that the enzyme is functional or active (e.g., can cleave the N-glycosidic bond between a uracil base and the sugar backbone in a DNA) at an elevated temperature, e.g., above 45°C, preferably above 50°C, more preferably above 55°C, more preferably above 60°C, even more preferably above 65°C, most preferably above 70°C, most preferably above 75°C, most preferably above 80°C, most preferably above 85°C, most preferably above 90°C, and even most preferably above 95°C. In some embodiments, the uracil-DNA N-glycosylase displays an optimum activity at one of the temperatures indicated above, e.g., the enzyme's temperature optimum is at one of the temperatures indicated above. The temperature stability of a uracil-DNA N-glycosylase can be increased to some extent by way of formulation of the composition comprising the uracil-DNA N-glycosylase, e.g., by combination with stabilizing chemicals or by immobilization of the enzyme, or by chemical modification, e.g., cross-linking, to preserve the enzyme in its active three dimensional shape.

As used herein, a "heat-stable" or "thermostable" enzyme remains active after at least 15 minutes, preferably for at least 2 hours, more preferably for at least 16 hours, more preferably for at least 24 hours, more preferably for at least 7 days, more preferably for at least 10 days, even more preferably for at least 14 days, most preferably for at least 30 days, even most preferably for at least 50 days at the elevated temperature and/or at the temperature of optimal activity. Generally, the level of activity is measured using an assay to measure the release of uracil from double-stranded, uracil-containing DNA, e.g., by measuring or monitoring the release of [³H]-uracil from DNA. For example, a definition for a "unit" of activity of a heat-stable uracil-DNA N-glycosylase is the amount of heat-stable uracil-DNA N-glycosylase that catalyzes the release of 60 pmol of uracil per minute from double-stranded, uracil-containing DNA, e.g., in a 50 µl reaction containing 0.2 µg DNA (e.g., at 10⁴-10⁵ cpm/µg) in 30 minutes at 65°C. The activity may be compared with the enzyme activity prior to the temperature elevation, thereby obtaining the residual activity of the enzyme or the activity retained by the enzyme after the heat treatment. Preferably, the residual activity is at least 30% after the given time at the elevated temperature, more preferably at least 40%, more preferably at least 50%, more preferably at least 60%, even more preferably at least 70%, most preferably at least 80%, even most preferably the residual activity is at least 90%, and absolutely most preferred the level of residual activity is at least equal to or unchanged after the given time at the elevated temperature. As such, providing "1 U" or "1 unit" of the enzyme refers to providing an amount of the enzyme (in combination with any other components such as a buffer, glycerol, etc. that accompany the enzyme) that catalyzes the release of 60 pmol of uracil per minute from double-stranded, uracil-containing DNA, e.g., in a 50 µl reaction containing 0.2 µg DNA (e.g., at 10⁴-10⁵ cpm/µg) in 30 minutes at 65°C or that would catalyze the release of 60 pmol of uracil per minute from double-stranded, uracil-containing DNA, e.g., if it were added to a 50 µl reaction containing 0.2 µg DNA (e.g., at 10⁴-10⁵ cpm/µg) and incubated for 30 minutes at 65°C, whether or not the enzyme is added to a 50 µl reaction containing 0.2 µg DNA (e.g., at 10⁴-10⁵ cpm/µg) and incubated for 30 minutes at 65°C.

As used herein, the term "active" or "activity" when referring to a UDG means the UDG cleaves the N-glycosidic bond between a uracil base and the sugar backbone in a DNA at some physiologically relevant and detectable level.

### Embodiments of the technology

Embodiments of the technology relate to methods for processing a sample comprising a nucleic acid in which the sample is heated, e.g., to 40°C, 50°C, 60°C, 70°C, 80°C, 90°C, 95°C, 97°C or more, e.g., for 2 minutes, 5 minutes, 10 minutes, 20 minutes, 30 minutes, 60 minutes or more. In particular, the technology relates to adding a uracil-DNA N-glycosylase to such samples to minimize or eliminate the subsequent detection of sequence errors caused by thermal deamination of cytosines during the period of heating.

One common example in which a sample comprising a nucleic acid is heated to these temperatures is the use of "hot-start PCR" to minimize nonspecific primer interactions with templates and the significant activity that thermophilic polymerases have at ambient temperatures (e.g., AmpliTaq Gold™ DNA polymerase; see, e.g., U.S. Pat. Nos. 5,773,258; 6,183,998). These methods used a thermostable polymerase, typically a Taq DNA polymerase, that is inactive at temperatures near ambient (room) temperature but that is active at higher temperatures. In particular, the thermostable polymerase is chemically cross-linked to inactivate the enzyme. The nature of the crosslinkers and the chemical bonds formed in these methods are reversible and the cross-linked thermostable polymerase is reactivated by heating the polymerase prior to the reaction for a predetermined amount of time at 95°C. Other hot-start PCR enzymes are inactivated by antibodies or nucleic acid aptamers that bind to and inhibit the polymerase at low temperatures but are released from the active enzyme at higher temperatures. The technology disclosed herein is related in some aspects to the use of heat activated polymerases in PCR and the use of a thermostable UDG in the sample to minimize generation of and detection of sequence errors resulting from deamination of cytosines during the high-temperature incubation.

Thus, in some embodiments, the technology comprises the use of a UDG that has activity at 40°C, 50°C, 60°C, 70°C, 80°C, 90°C, 95°C, or 97°C or more during and after exposure to that temperature for 2 minutes, 5 minutes, 10 minutes, 20 minutes, 30 minutes,or 60 minutes or more. In some embodiments, the UDG is an enzyme isolated from a thermophilic organism such as a thermophilic member of the Archaea or Bacteria. In some embodiments, the UDG is variant of a mesophilic UDG comprising amino acid substitutions that confer a higher thermostability relative to the wild-type UDG. In some embodiments, the UDG has been produced by random mutation, rational modeling and design, or in vitro evolution. The technology contemplates use of a thermostable UDG regardless of its source.

In some embodiments, the UDG is present in a sample comprising a nucleic acid before the addition of a polymerase and high-temperature incubation of the polymerase (e.g., to activate it). The sample comprising the UDG and nucleic acid is incubated at a temperature at which the UDG is active (e.g., 65°C) to remove the U bases from the nucleic acid that may be present prior to the high-temperature activation of the polymerase. In some embodiments, the UDG is present in the sample comprising a nucleic acid and a polymerase during the high-temperature incubation to activate the polymerase.

The technology relates to removing U bases from DNA prior to amplification of DNA. This cleavage generates abasic sites that are blocked from replication by DNA polymerase. As a result, the polymerase does not replicate the damaged DNA strand and the C to U mutation is not propagated in the population of amplicons during PCR amplification. Although the disclosure herein refers to certain illustrated embodiments, it is to be understood that these embodiments are presented by way of example and not by way of limitation. Experimental examples are provided to describe exemplary embodiments of the technology.

Some embodiments of the technology encompass compositions, methods, uses, kits, and systems related to an enzyme or enzymatic activity that produces a break in a nucleic acid at an abasic site, e.g., an abasic site produced by an enzyme that removes uracil from DNA. In some embodiments, the enzyme that causes a break in a nucleic acid at an abasic site is a thermostable enzyme. In some embodiments, the enzyme is an endonuclease that cleaves DNA at an abasic site, e.g., a thermostable apurinic/apyrimidinic endonuclease from *Thermus thermophilus* such as a *Tth* Endo IV, e.g., as supplied by New England Biolabs. Such an enzyme hydrolyzes DNA at an abasic site at the first phosphodiester bond 5' to the lesion leaving a 3' hydroxyl and a deoxyribose 5'-phosphate at the 5' terminus. Some enzymes also have a 3'-diesterase activity. Furthermore, in some embodiments both the N-glycosylase and the cleavage activities are provided by a single enzyme. For example, in some embodiments, the enzyme is a homolog of *E. coli* Endonuclease III (Nth). In some embodiments, the enzyme is thermostable. This enzyme has both an N-glycosylase and a lyase (cleavage) activity. The N-glycosylase activity releases damaged pyrimidines from DNA (e.g., a deaminated cytosine), generating an abasic site; then, the lysase activity cleaves the resulting abasic site to produce a break in the nucleic acid strand. The enzyme also recognizes and cleaves abasic sites that do not result from its N-glycosylase activity (e.g., abasic sites produced by an enzyme that removes uracil from DNA, e.g., a UDG). In some embodiments, the enzyme is isolated from a thermophile such as *Thermotoga maritima,* e.g., *Tma* Endonuclease III as provided by New England Biolabs.

### Examples

### Identification of mutations in KRAS

During the development of embodiments of the technology disclosed herein, experiments were performed to test a heat stable UDG enzyme to reduce the incidence of errors in detecting KRAS mutants by PCR. Mutations in KRAS are associated with human cancers and thus KRAS is a target of many cancer diagnostics. In particular, some cancers are associated with mutations that introduce an amino acid substitution at position 12 or 13 of KRAS, which are glycine residues encoded by the codons GGT and GGC in the wild-type KRAS gene sequence. Prevalent substitutions that result from mutations in the KRAS gene include a mutation of the wild-type G at position 35 to an A (c.35G>A) in the KRAS gene that results in a substitution of the wild-type glycine at position 12 to an aspartic acid (p.G12D) in the KRAS protein; a mutation of the wild-type G at position 38 to an A (c.38G>A) in the KRAS gene that results in a substitution of the wild-type glycine at position 13 to an aspartic acid (p.G13D) in the KRAS protein; a mutation of the wild-type G at position 34 to an A (c.34G>A) in the KRAS gene that results in a substitution of the wild-type glycine at position 12 to a serine (p.G12S) in the KRAS protein; and a mutation of the wild-type G at position 37 to an A (c.37G>A) in the KRAS gene that results in a substitution of the wild-type glycine at position 13 to a serine (p.G13S) in the KRAS protein. Other mutations and substitutions are known; the experiment focused on detecting G12D, G13D, G12S, and G13S.

These mutations thus occur due to a G to A mutation in the coding strand or due to a C to T mutation in the non-coding strand. While C to T mutations occur naturally and may be present in a sample, they are also produced from a wild-type sample by thermal deamination of the C residue opposite the G at position 34, 35, 37, or 38 in the KRAS coding sequence. As such, thermal deamination produces false positive results that mutant KRAS sequences are present in wild-type samples. Such a result can result in a false cancer diagnosis.

Accordingly, experiments were conducted to test the hypothesis that heat-induced deamination of C to U generates single copies of G-to-A KRAS mutant gene sequences in samples containing only wild-type targets. Mutant amplicons generated from these single copies in a wild-type sample are detected by a G13D allele-specific probe and thus produce a signal in a wild-type sample that is comparable to a signal produced by a G13D mutant sample. Four major observations support this hypothesis:
1) The incidence of false positives in wild-type samples is proportional to input copy number. For example, a series of 24 samples each comprising 6000 copies of a wild-type sequence results in approximately 10 of the 24 samples testing falsely positive for KRAS mutations while the same experiment using samples comprising 600 copies of the wild-type sequence in each sample results in approximately 1 of the 24 wells testing falsely positive for mutant KRAS sequences. This rate of false positive occurrence is consistent with the published rate of cytosine deamination at 95°C (Lindahl and Nyberg, supra);
2) the Cₜ of detecting a false positive in a real-time PCR is consistent with what is expected for amplification from a single copy;
3) DNA sequencing of samples testing falsely positive for mutant KRAS identifies the mutant sequence; and
4) activation of Taq polymerase apart from the sample comprising DNA target (such that target is not incubated at 95°C) greatly reduces the incidence of false positives.

Based on these data, experiments were performed to test a heat-stable uracil-DNA-N-glycosylase (UDG) in improving assay specificity/sensitivity.

Experiments used PCR to amplify the region of the KRAS gene comprising the G12 and G13 codons from input template DNA comprising a wild-type KRAS sequence or a mutant KRAS sequence. Labeled probes specific for the mutant sequences were then used to detect the presence of the mutant sequences in the amplified samples.

Experiments used real-time PCR and MaxRatio analysis as described in Shain and Clements (2008), "A new method for robust quantitative and qualitative analysis of real-time PCR" Nucl. Acids Res. 36: e91. In the real-time PCR plots shown in panels A and C of Figures 1 through 3, the x-axis depicts the PCR cycle number and the y-axis depicts relative fluorescence units. In the MaxRatio analyses plots shown in panels B and D of Figures 1 through 3, The x-axis represents cycle number (analogous to Ct) and the y-axis depicts the MaxRatio. Each replicate for each experiment is individually plotted on the figures. Experiments with 100% wild-type
KRAS sequence are indicated with arrow heads and experiments with 1% mutant KRAS sequence are indicated with arrows.

In the first experiment, 42 replicates of 5 ng of human genomic DNA containing 100% wild-type KRAS sequence and 6 replicates of 5 ng of human genomic DNA containing 1% G12D mutant KRAS sequence were assayed by real-time PCR using a G12D specific probe for the presence of the G12D mutant sequence. The experiment was performed in the absence of UDG (Figures 1A; 1B) and in the presence of 2 units of UDG (Figures 1C; 1D).

In the second experiment, 42 replicates of 5 ng of human genomic DNA containing 100% wild-type KRAS sequence and 6 replicates of 5 ng of human genomic DNA containing 1% G13D mutant KRAS sequence were assayed by real-time PCR using a G13D specific probe for the presence of the G13D mutant sequence. The experiment was performed in the absence of UDG (Figures 2A; 2B) and in the presence of 2 units of UDG (Figures 2C; 2D).

In the third experiment, 12 replicates ranging from 50 ng to 400 ng of human genomic DNA containing 100% wild-type KRAS sequence and 12 replicates ranging from 50 ng to 400 ng of human genomic DNA containing 1% G12D mutant KRAS sequence were assayed by real-time PCR using a G12D specific probe for the presence of the G12D mutant sequence. The experiment was performed in the absence of UDG (Figures 3A; 3B) and in the presence of 2 units of UDG (Figures 3C; 3D).

Experiments used a heat-stable UDG, such as *Afu* Uracil-DNA glycosylase (UDG) from New England Biolabs, which is a thermostable homolog of the *E. coli* Uracil-DNA glycosylase isolated from *Archaeglobus fulgidis.* An amount of 50% glycerol was added to samples without UDG that was equivalent to the amount of glycerol added to the samples to which UDG enzyme in glycerol was added. Thermocycling conditions were: 1 cycle of 93.5°C for 10 minutes; 1 cycle of 73.5°C for 10 minutes; 3 cycles of 92.0°C for 15 seconds, 73.5°C for 30 seconds, and 61.0°C for 60 seconds; followed by 45 cycles of 92°C for 15 seconds and 61°C for 90 seconds.

Experiments demonstrated that a heat-stable UDG (e.g., 2 units of a heat-stable UDG) reduced the incidence of false positives and delayed the Cₜ of false positive events that did occur. To test the activity of UDG further, UDG was evaluated under a variety of conditions to assess if the specificity and/or sensitivity of detecting KRAS wild-type and mutant sequences were improved in the presence of a heat-stable uracil-DNA-N-glycosylase.

Data collected during the development of embodiments of the technology disclosed herein demonstrated that the incidence of false positives is greatly reduced by including UDG in the assay samples. These data demonstrate that including a heat-stable UDG in the samples enhanced assay results. These data demonstrated that false positives were eliminated or minimized; false positives were detected at a considerably higher Cₜ value, thereby providing enhanced assay specificity.

Various modifications and variations of the described compositions, methods, and uses of the technology will be apparent to those skilled in the art.

## Claims

1. A composition comprising a target nucleic acid comprising: a) a target sequence, b) a heat-activated polymerase, c) at least one of: i) a heat stable enzyme that removes uracil from DNA and cleaves DNA at an abasic site; ii) a heat stable enzyme that removes uracil from DNA and an enzyme that cleaves DNA at an abasic site or iii) a heat stable uracil-DNA glycosylase, and d) a damaged nucleic acid comprising a uracil base.

2. The composition of claim 1 further comprising a probe specific for the target sequence.

3. The composition of any of claims 1 and 2 wherein the heat-activated polymerase is Taq polymerase.

4. The composition of any of claims 1 and 3 comprising an amount or concentration of the uracil-DNA glycosylase that is sufficient to remove uracil from DNA during and/or after a period of heat-activation of a heat-activated polymerase at a rate that is at least 30% of a rate at which the uracil-DNA glycosylase removes bases prior to the period of heat-activation of the heat-activated polymerase.

5. A kit for detecting a nucleic acid, the kit comprising:
a) a first vessel comprising a heat-activated polymerase; and a second vessel comprising a thermostable uracil-DNA glycosylase;
b) a vessel comprising a heat-activated polymerase and a thermostable uracil-DNA glycosylase;
c) a first vessel comprising a heat-activated polymerase; and a second vessel comprising a heat stable enzyme that removes uracil from DNA;
d) a first vessel comprising a heat-activated polymerase; and a second vessel comprising a heat stable enzyme that removes uracil from DNA and that cleaves DNA at an abasic site;
e) a first vessel comprising a heat-activated polymerase; a second vessel comprising a heat stable enzyme that removes uracil from DNA; and a third vessel comprising an enzyme that cleaves DNA at an abasic site; or
f) a first vessel comprising a heat-activated polymerase; and a second vessel comprising a heat stable enzyme that removes uracil from DNA and an enzyme that cleaves DNA at an abasic site;
wherein the kit further comprises a control nucleic acid, wherein the control nucleic acid is a positive control nucleic acid and/or an internal reference control, wherein said internal reference control comprises synthetic DNA molecules comprising one or more uracil bases.

6. A method for detecting a target nucleic acid comprising a target sequence, the method comprising:
a) providing a sample comprising the target nucleic acid;
b) providing a positive control nucleic acid comprising one or more uracil bases;
c) adding at least one of: i) a heat stable enzyme that removes uracil from DNA and cleaves DNA at an abasic site; ii) a heat stable enzyme that removes uracil from DNA and an enzyme that cleaves DNA at an abasic site or iii) a heat stable uracil-DNA glycosylase;
d) excising a uracil base from:
i) the positive control nucleic acid; and
ii) from a damaged target nucleic acid, if present;
e) thermocycling the reaction mixture to produce:
i) an amplicon comprising the target sequence; and
ii) an amplicon comprising the positive control nucleic acid sequence; and
f) detecting the amplicon comprising the target sequence
wherein the damaged nucleic acid is not amplified, is not detected, and/or is amplified less than the target nucleic acid, if present.

## Patentansprüche

1. Zusammensetzung, die eine Zielnukleinsäure umfasst, umfassend: a) eine Zielsequenz, b) eine hitzeaktivierte Polymerase, c) mindestens eines aus: i) einem hitzestabilen Enzym, das Uracil aus DNA entfernt und DNA an einer abasischen Stelle spaltet; ii) einem hitzestabilen Enzym, das Uracil aus DNA entfernt, und einem Enzym, das DNA an einer abasischen Stelle spaltet, oder iii) einer hitzestabilen Uracil-DNA-Glykosylase, und d) eine beschädigte Nukleinsäure, die eine Uracilbase umfasst.

2. Zusammensetzung nach Anspruch 1, die ferner eine für die Zielsequenz spezifische Sonde umfasst.

3. Zusammensetzung nach einem der Ansprüche 1 und 2, wobei es sich bei der hitzeaktivierten Polymerase um Taq-Polymerase handelt.

4. Zusammensetzung nach einem der Ansprüche 1 und 3, die eine Menge oder Konzentration der Uracil-DNA-Glykosylase umfasst, die ausreicht, um Uracil aus DNA während und/oder nach einem Zeitraum der Hitzeaktivierung einer hitzeaktivierten Polymerase mit einer Rate zu entfernen, die mindestens 30% einer Rate beträgt, mit der die Uracil-DNA-Glykosylase vor dem Zeitraum der Hitzeaktivierung der hitzeaktivierten Polymerase Basen entfernt.

5. Kit zum Nachweisen einer Nukleinsäure, wobei das Kit Folgendes umfasst:
a) ein erstes Gefäß, das eine hitzeaktivierte Polymerase umfasst, und ein zweites Gefäß, das eine thermostabile Uracil-DNA-Glykosylase umfasst;
b) ein Gefäß, das eine hitzeaktivierte Polymerase und eine thermostabile Uracil-DNA-Glykosylase umfasst;
c) ein erstes Gefäß, das eine hitzeaktivierte Polymerase umfasst, und ein zweites Gefäß, das ein hitzestabiles Enzym, das Uracil aus DNA entfernt, umfasst;
d) ein erstes Gefäß, das eine hitzeaktivierte Polymerase umfasst, und ein zweites Gefäß, das ein hitzestabiles Enzym, das Uracil aus DNA entfernt und das DNA an einer abasischen Stelle spaltet, umfasst;
e) ein erstes Gefäß, das eine hitzeaktivierte Polymerase umfasst, ein zweites Gefäß, das ein hitzestabiles Enzym, das Uracil aus DNA entfernt, umfasst, und ein drittes Gefäß, das ein Enzym, das DNA an einer abasischen Stelle spaltet, umfasst; oder
f) ein erstes Gefäß, das eine hitzeaktivierte Polymerase umfasst, und ein zweites Gefäß, das ein hitzestabiles Enzym, das Uracil aus DNA entfernt, und ein Enzym, das DNA an einer abasischen Stelle spaltet, umfasst,
wobei das Kit ferner eine Kontrollnukleinsäure umfasst, wobei die Kontrollnukleinsäure eine positive Kontrollnukleinsäure und/oder eine interner-Standard-Kontrolle ist, wobei die interner-Standard-Kontrolle synthetische DNA-Moleküle umfasst, die eine oder mehrere Uracilbasen umfassen.

6. Verfahren zum Nachweisen einer Zielnukleinsäure, die eine Zielsequenz umfasst, wobei das Verfahren Folgendes umfasst:
a) Bereitstellen einer Probe, die die Zielnukleinsäure umfasst;
b) Bereitstellen einer positiven Kontrollnukleinsäure, die eine oder mehrere Uracilbasen umfasst;
c) Hinzufügen von mindestens einem aus: i) einem hitzestabilen Enzym, das Uracil aus DNA entfernt und DNA an einer abasischen Stelle spaltet; ii) einem hitzestabilen Enzym, das Uracil aus DNA entfernt, und einem Enzym, das DNA an einer abasischen Stelle spaltet, oder iii) einer hitzestabilen Uracil-DNA-Glykosylase;
d) Ausschneiden einer Uracilbase aus:
i) der positiven Kontrollnukleinsäure und
ii) aus der beschädigten Nukleinsäure, falls vorhanden;
e) Thermozyklisieren des Reaktionsgemischs, um:
i) ein Amplikon herzustellen, das die Zielsequenz umfasst, und
ii) ein Amplikon herzustellen, das die positive Kontrollnukleinsäuresequenz umfasst; und
f) Nachweisen des Amplikons, das die Zielsequenz umfasst,
wobei die beschädigte Nukleinsäure nicht amplifiziert wird, nicht nachgewiesen wird und/oder weniger amplifiziert wird als die Zielnukleinsäure, falls vorhanden.

## Revendications

1. Composition comprenant un acide nucléique cible qui comprend : a) une séquence cible, b) une polymérase activée par la chaleur, c) au moins l'un des éléments suivants : i) une enzyme thermostable qui ôte l'uracile de l'ADN et coupe l'ADN au niveau d'un site abasique ; ii) une enzyme thermostable qui ôte l'uracile de l'ADN et une enzyme qui coupe l'ADN au niveau d'un site abasique ou iii) une uracile-ADN glycosylase thermostable, et d) un acide nucléique endommagé comprenant une base d'uracile.

2. Composition de la revendication 1 comprenant en outre une sonde spécifique de la séquence cible.

3. Composition de l'une quelconque des revendications 1 et 2, dans laquelle la polymérase activée par la chaleur est une Taq polymérase.

4. Composition de l'une quelconque des revendications 1 et 3 comprenant une quantité ou concentration de l'uracile-ADN glycosylase qui est suffisante pour ôter l'uracile de l'ADN pendant et/ou après une période d'activation par la chaleur d'une polymérase activée par la chaleur, à un taux qui est au moins 30% d'un taux auquel l'uracile-ADN glycosylase ôte des bases avant la période d'activation par la chaleur de la polymérase activée par la chaleur.

5. Trousse destinée à détecter un acide nucléique, la trousse comprenant :
a) un premier récipient comprenant une polymérase activée par la chaleur ; et un deuxième récipient comprenant une uracile-ADN glycosylase thermostable ;
b) un récipient comprenant une polymérase activée par la chaleur et une uracile-ADN glycosylase thermostable ;
c) un premier récipient comprenant une polymérase activée par la chaleur ; et un deuxième récipient comprenant une enzyme thermostable qui ôte l'uracile de l'ADN ;
d) un premier récipient comprenant une polymérase activée par la chaleur ; et un deuxième récipient comprenant une enzyme thermostable qui ôte l'uracile de l'ADN et qui coupe l'ADN au niveau d'un site abasique ;
e) un premier récipient comprenant une polymérase activée par la chaleur ; un deuxième récipient comprenant une enzyme thermostable qui ôte l'uracile de l'ADN ; et un troisième récipient comprenant une enzyme qui coupe l'ADN au niveau d'un site abasique ; ou
f) un premier récipient comprenant une polymérase activée par la chaleur ; et un deuxième récipient comprenant une enzyme thermostable qui ôte l'uracile de l'ADN et une enzyme qui coupe l'ADN au niveau d'un site abasique ;
la trousse comprenant en outre un acide nucléique témoin, dans laquelle l'acide nucléique témoin est un acide nucléique témoin positif et/ou un témoin de référence interne, dans laquelle ledit témoin de référence interne comprend des molécules d'ADN de synthèse comprenant une ou plusieurs base(s) d'uracile.

6. Procédé destiné à détecter un acide nucléique cible comprenant une séquence cible, le procédé comprenant les étapes consistant à :
a) fournir un échantillon comprenant l'acide nucléique cible ;
b) fournir un acide nucléique témoin positif comprenant une ou plusieurs base(s) d'uracile ;
c) ajouter au moins l'un des éléments suivants :
i) une enzyme thermostable qui ôte l'uracile de l'ADN et coupe l'ADN au niveau d'un site abasique ; ii) une enzyme thermostable qui ôte l'uracile de l'ADN et une enzyme qui coupe l'ADN au niveau d'un site abasique ou iii) une uracile-ADN glycosylase thermostable ;
d) exciser une base d'uracile à partir :
i) de l'acide nucléique témoin positif ; et
ii) à partir d'un acide nucléique cible endommagé, s'il est présent ;
e) effectuer un thermo-cyclage du mélange réactionnel pour produire :
i) un amplicon comprenant la séquence cible ; et
ii) un amplicon comprenant la séquence d'acide nucléique témoin positif ; et
f) détecter l'amplicon comprenant la séquence cible
dans lequel l'acide nucléique endommagé n'est pas amplifié, n'est pas détecté et/ou est moins amplifié que l'acide nucléique cible, s'il est présent.
